# EUROPÄISCHE PATENTSCHRIFT

(11) **EP 1 200 615 B1**
(45) Veröffentlichungstag und Bekanntmachung des Hinweises auf die Patenterteilung: **25.04.2007**
(21) Anmeldenummer: 00958396.4
(22) Anmeldetag: 08.08.2000
(51) Int. Cl.: C12N 15/82, C12N 9/10, C12N 5/10, A23L 1/05, A01H 5/00, A23L 1/0522

(54) **NUKLEINSÄUREMOLEKÜLE AUS PFLANZEN CODIEREND ENZYME, DIE AN DER STÄRKESYNTHESE BETEILIGT SIND**
NUCLEIC ACID MOLECULES DERIVED FROM PLANTS WHICH CODE FOR ENZYMES WHICH ARE INVOLVED IN THE SYNTHESIS OF STARCH
MOLECULES D'ACIDE NUCLEIQUE EXTRAITES DE PLANTES CODANT DES ENZYMES QUI PARTICIPENT A LA SYNTHESE DE L'AMIDON

(30) Priorität: 11.08.1999 DE 19937348
(43) Veröffentlichungstag der Anmeldung: 02.05.2002
(73) Patentinhaber: Bayer CropScience GmbH, 65929 Frankfurt/Main (DE)
(72) Erfinder: FROHBERG, Claus, 14532 Kleinmachnow (DE)
(74) Vertreter: Vossius & Partner
(86) Internationale Anmeldenummer: PCT/EP2000/007673
(87) Internationale Veröffentlichungsnummer: WO 2001/012826

(56) Entgegenhaltungen:
- EP-A- 0 779 363
- WO-A-96/15248
- WO-A-97/26362
- WO-A-99/24575
- DATABASE EMBL [Online] ACCESSION NO: C99345, 16. Oktober 1998 (1998-10-16) SASAKI T.: "Oryza sativa cDNA, partial sequence (E10736_8Z)." XP002163786
- DATABASE EMBL [Online] ACCESSION NO: AI947842, 23. August 1999 (1999-08-23) WALBOT V.: "603029D11.x1 603 - stressed root cDNA library from Wang/Bohnert lab Zea mays cDNA, mRNA sequence." XP002163787
- CAO HEPING ET AL: "Identification of the soluble starch synthase activities of maize endosperm." PLANT PHYSIOLOGY (ROCKVILLE), Bd. 120, Nr. 1, Mai 1999 (1999-05), Seiten 205-215, XP002163785 ISSN: 0032-0889 in der Anmeldung erwähnt

## Beschreibung

Die vorliegende Erfindung betrifft Nukleinsäuremoleküle, die ein Enzym codieren, das an der Stärkesynthese in Pflanzen beteiligt ist. Bei diesem Enzym handelt es sich um eine neue Isoform der Stärkesynthase.
Weiterhin betrifft diese Erfindung Vektoren, sowie mit den beschriebenen Nukleinsäuremolekülen oder Vektoren transformierte Wirtszellen, insbesondere Pflanzenzellen und aus diesen regenerierbare Pflanzen.
Femer werden Verfahren zur Herstellung transgener Pflanzenzellen und Pflanzen beschrieben, die aufgrund der Einführung von DNA-Molekülen, die eine Stärkesynthase codieren, eine in ihren Eigenschaften veränderte Stärke synthetisieren. Die vorliegende Erfindung betrifft auch die aus den erfindungsgemäßen Pflanzenzellen und Pflanzen erhältliche Stärke sowie Verfahren zur Herstellung dieser Stärke.

Im Hinblick auf die zunehmende Bedeutung, die pflanzlichen Inhaltsstoffen als erneuerbaren Rohstoffquellen in letzter Zeit beigemessen wird, ist es eine der Aufgaben der biotechnologischen Forschung, sich um eine Anpassung dieser pflanzlichen Rohstoffe an die Anforderungen der verarbeitenden Industrie zu bemühen. Um eine Anwendung von nachwachsenden Rohstoffen in möglichst vielen Einsatzgebieten zu ermöglichen, ist es darüber hinaus erforderlich, eine große Stoffvielfalt zu erreichen.
Neben Ölen, Fetten und Proteinen stellen Polysaccharide die wesentlichen nachwachsenden Rohstoffe aus Pflanzen dar. Eine zentrale Stellung bei den Polysacchariden nimmt neben Zellulose die Stärke ein, die einer der wichtigsten Speicherstoffe in höheren Pflanzen ist. Hierbei ist Mais eine der interessantesten Pflanzen, da sie weltweit für die Stärkeproduktion die wichtigste Kulturpflanze ist.

Das Polysaccharid Stärke ist ein Polymer aus chemisch einheitlichen Grundbausteinen, den Glucosemolekülen. Es handelt sich dabei jedoch um ein sehr komplexes Gemisch aus unterschiedlichen Molekülformen, die sich hinsichtlich ihres Polymerisationsgrades und des Auftretens von Verzweigungen der Glucoseketten unterscheiden. Daher stellt Stärke keinen einheitlichen Rohstoff dar. Man unterscheidet insbesondere die Amylose-Stärke, ein im wesentlichen unverzweigtes Polymer aus α-1,4-glycosidisch verknüpften Glucosemolekülen, von der Amylopektin-Stärke, die ihrerseits ein komplexes Gemisch aus unterschiedlich verzweigten Glucoseketten darstellt. Die Verzweigungen kommen dabei durch das Auftreten von zusätzlichen α-1,6-glycosidischen Verknüpfungen zustande. In typischen für die Stärkeproduktion verwendeten Pflanzen, wie z. B. Mais oder Kartoffel, besteht die synthetisierte Stärke zu ca. 20 % - 25 % aus Amylose-Stärke und zu ca. 75 % - 80 % aus Amylopektin-Stärke.
Um eine möglichst breite Anwendung von Stärke zu ermöglichen, erscheint es wünschenswert, Pflanzen zur Verfügung zu stellen, die in der Lage sind, modifizierte Stärke zu synthetisieren, die sich für verschiedene Verwendungszwecke besonders eignet. Eine Möglichkeit, derartige Pflanzen bereitzustellen, besteht - neben züchterischen Maßnahmen - in der gezielten genetischen Veränderung des Stärkemetabolismus stärkeproduzierender Pflanzen durch gentechnolögische Methoden. Voraussetzung hierfür ist jedoch die Identifizierung und Charakterisierung der an der Stärkesynthese und/oder -modifikation beteiligten Enzyme sowie die Isolierung der entsprechenden, diese Enzyme codierende DNA-Moleküle.
Die biochemischen Synthesewege, die zum Aufbau von Stärke führen, sind im wesentlichen bekannt. Die Stärkesynthese in pflanzlichen Zellen findet in den Plastiden, statt. In photosynthetisch aktiven Geweben sind dies die Chloroplasten, in photosynthetisch inaktiven, stärkespeichernden Geweben die Amyloplasten.
Die wichtigsten an der Stärkesynthese beteiligten Enzyme sind die Stärkesynthasen sowie die Verzweigungsenzyme. Bei den Stärkesynthasen sind verschiedene Isoformen beschrieben, die alle eine Polymerisierungsreaktion durch Übertragung eines Glucosylrestes von ADP-Glucose auf α-1,4-Glucane katalysieren.
Verzweigungsenzyme katalysieren die Einführung von α-1,6-Verzweigungen in lineare α-1,4-Glucane.
Stärkesynthasen können in zwei Klassen eingeteilt werden: die Stärkekomgebundenen Stärkesynthasen ("granule-bound starch synthases"; GBSS) und die löslichen Stärkesynthasen ("soluble starch synthases"; SS). Diese Unterscheidung ist nicht in jedem Fall eindeutig zu treffen, da einige der Stärkesynthasen sowohl stärkekomgebunden als auch in löslicher Form vorliegen (Denyer et al., Plant J. 4 (1993), 191-198; Mu et al., Plant J. 6 (1994), 151-159).
Basierend auf cDNA- und Aminosäuresequenzvergleichen wurden für Maispflanzen bisher, neben der Klasse der stärkekomgebundenen Stärkesynthasen, GBSSI, innerhalb der Klasse der löslichen Stärkesynthasen wiederum mindestens drei verschiedene Isoformen beschrieben.
Zur Isoform 1 der Stärkesynthase (= SSI) gehören Gene, die in Mais für ein ca. 76 kDa Protein zSSI (Mu et al., Plant J.6, (1994), 151-159) codieren, und die bisher nur für monokotyledone Pflanzen, wie z.B. für Reis (Baba et al., Plant Physiol. 103, (1993), 565-573), beschrieben worden und hauptsächlich im Endosperm exprimiert sind. Diese Proteine werden in der Regel durch Citrat stimuliert und sind unabhängig von sogenannten Primer-Molekülen.
Im Gegensatz dazu sind Stärkesynthasen der Isoform II (= SSII) in der Regel abhängig von Primermolekülen und zeigen die höchste Sequenzhomologie zu den SSII-Isoformen, die früher teilweise als GBSSII bezeichnet worden sind, aus Erbse (Dry et al., Plant J. 2, (1992), 193-202) und aus Kartoffel (Edwards et al., Plant J 8, . (1995), 283-294).
Bei Betrachtung der SSII aus Mais muß unterschieden werden zwischen den Genen bzw. cDNAs, die in der Literatur als zSSlla und zSSllb bezeichnet werden (Ham et al., Plant Mol. Biol. 37, (1998), 639-649; Imparl-Radosevich, Arch. Biochem.
Biophys. 362, (1999), 131-138), und dem sogenannten SSII-Protein, einem ca. 180 kDa-Protein (Molekulargewicht bestimmt mittels Gelfiltration (Mu et al., Plant J. 6, (1994), 151-159)) aus Mais-Endosperm, dessen Name auf früheren biochemischen Untersuchungen basiert (Boyer and Preiss, Plant Physiol. 67, (1981), 1141-1145; Mu et al., Plant J. 6, (1994), 151-159). Momentan ist die Frage, welches Gen mit diesem 180 kDa-Protein tatsächlich korrespondiert, nicht abschließend geklärt (Imparl-Radosevich, Arch. Biochem. Biophys. 362, (1999), 131-138). Cao et al. (Plant Physiol. 120, (1999), 205-215) schlagen das sogenannte du1-Gen als korrespondierendes Gen zum 180-kDa-Protein vor.
Die dritte bisher beschriebene Klasse an Stärkesynthase-Genen; die als SSIII bezeichnet werden, codieren in Kartoffeln ein 139-kDa-Protein (Abel et al., Plant J. 10, (1996), 981-991; Marshall et al., Plant Cell 8, (1996), 1121-1135), das in Kartoffelknollen 80% der gesamten Stärkesynthaseaktivität ausmacht. Aufgrund der hohen Konservierung bestimmter Sequenzbereiche des C-Terminus im Vergleich zur Kartoffel-SSIII-Aminosäuresequenz wurde für das ursprünglich als du1-Gen bezeichnete Gen aus Mais die Umbenennung in zSSlll vorgeschlagen (Cao et al., Plant Physiol. 120, (1999), 205-215), wobei der Präfix "z" den Ursprungsorganismus Zea mays bezeichnet.
Lediglich für die Isoform GBSS I gelang es bisher, die genaue Funktion bei der Stärkesynthese zu ermitteln. Pflanzen, in denen diese Enzymaktivität stark oder vollkommen reduziert ist, synthetisieren eine amylosefreie (sogenannte "waxy") Stärke (Shure et al., Cell35 (1983), 225-233; Visser et al., Mol. Gen: Genet. 225 (1991), 289-296; WO9211376A1), so daß diesem Enzym eine entscheidende Rolle bei der Synthese der Amylose-Stärke zugesprochen wird. Dieses Phänomen wird ebenfalls in Zellen der Grünalge *Chlamydomonas reinhardtii* beobachtet (Delrue et al., J. Bacteriol. 174 (1992), 3612-3620). Bei Chlamydomonas konnte darüber hinaus gezeigt werden, daß GBSS 1 nicht nur an der Synthese der Amylose beteiligt ist, sondern auch einen Einfluß auf die Amylopektinsynthese besitzt. In Mutanten, die keine GBSSt-Aktivität aufweisen, fehlt eine bestimmte Fraktion des normalerweise synthetisierten Amylopektins, die längerkettige Glucane aufweist.
Die Funktionen der Isoformen der löslichen Stärkesynthasen sind bisher unklar. Es wird angenommen, daß die löslichen Stärkesynthasen zusammen mit Verzweigungsenzymen an der Synthese des Amylopektins beteiligt sind (siehe z.B. Ponstein et al., Plant Physiol. 92 (1990), 234-241) und daß sie eine wichtige Funktion bei der Regulation der Stärkesyntheserate spielen.
Außer beim Mais wurden lösliche Stärkesynthasen auch in einer Reihe weiterer Pflanzenarten identifiziert. Lösliche Stärkesynthasen sind beispielsweise bis zur Homogenität aus Erbse (Denyer und Smith, Planta 186 (1992), 609-617) und Kartoffel (Edwards et al., Plant J. 8 (1995), 283-294) isoliert worden. In diesen Fällen stellte sich heraus, daß die als SS II identifizierte Isoform der löslichen Stärkesynthase identisch ist mit der Stärkekorn-gebundenen Stärkesynthase GBSS II (Denyer et al., Plant J. 4 (1993), 191-198; Edwards et al., Plant J. 8 (1995), 283-294). Für einige weitere Pflanzenspezies wurde das Vorhandensein mehrerer SS-Isoformen mit Hilfe chromatographischer Methoden beschrieben, beispielsweise bei Gerste (Tyynelä und Schulman, Physiologia Plantarum 89 (1993) 835-841; Kreis, Planta 148 (1980), 412-416) und Weizen (Rijven, Plant Physiol. 81 (1986), 448-453). DNA-Sequenzen, die diese Proteine codieren, wurden ebenfalls beschrieben (s. beispielsweise GenBank Acc. No. U48227; Vrinten et al., Mol. Gen. Genet. 261 (3), (1999), 463-471).

Um weitere Möglichkeiten bereitzustellen, beliebige stärkespeichernde Pflanzen dahingehend zu verändern, daß sie eine modifizierte Stärke synthetisieren, ist es erforderlich, jeweils DNA-Sequenzen zu identifizieren, die weitere Isoformen der Stärkesynthasen codieren.

Der vorliegenden Erfindung liegt somit die Aufgabe zugrunde, Nukleinsäuremoleküle zur Verfügung zu stellen, die an der Stärkebiosynthese beteiligte Enzyme codieren und mit deren Hilfe es möglich ist, gentechnisch veränderte Pflanzen herzustellen, die eine erhöhte oder erniedrigte Aktivität dieser Enzyme aufweisen, wodurch es zu einer Veränderung der chemischen und/oder physikalischen Eigenschaften der in diesen Pflanzen synthetisierten Stärke kommt und diese somit für allgemeine und/oder spezielle Verwendungszwecke besser geeignet ist.

Diese Aufgabe wird durch die Bereitstellung der in den Patentansprüchen bezeichneten Ausführungsformen gelöst.

Die vorliegende Erfindung betrifft daher Nukleinsäuremoleküle, die Proteine mit der biologischen Aktivität einer Stärkesynthase codieren oder ein biologisch aktives Fragment eines solchen Proteins, wobei derartige Moleküle vorzugsweise Proteine mit der unter Seq ID No. 2 angegebenen Aminosäuresequenz codieren.
Insbesondere betrifft die Erfindung Nukleinsäuremoleküle, die die unter Seq ID No. 1 angegebene Nucleotidsequenz oder einen Teil davon enthalten, bevorzugt Moleküle, die die in Seq ID No. 1 angegebene codierende Region umfassen bzw. entsprechende Ribonucleotidsequenzen.
Die Erfindung betrifft auch Nukleinsäuremoleküle, die eine Sequenz aufweisen, die zu der gesamten oder einem Teil der unter Seq ID No. 1 dargestellten Sequenz komplementär ist.
Gegenstand der Erfindung sind ebenfalls Nukleinsäuremoleküle, die eine Stärkesynthase oder ein biologisch aktives Fragment davon codieren und/oder deren Sequenz aufgrund der Degeneration des genetischen Codes von den Nucleotidsequenzen der oben beschriebenen Moleküle abweicht.
Ferner betrifft die vorliegende Erfindung Nukleinsäuremoleküle, die eine Stärkesynthase oder ein biologisch aktives Fragment davon codieren und die mit einem der oben beschriebenen Moleküle hybridisieren.

Bei den erfindungsgemäßen Nukleinsäuremolekülen kann es sich sowohl um DNAals auch RNA-Moleküle handeln. Entsprechende DNA-Moleküle sind beispielsweise genomische oder cDNA-Moleküle. RNA-Moleküle können beispielsweise mRNA oder antisense-RNA-Moleküle sein.
Die vorliegende Erfindung betrifft daher auch Nucleotidsequenzen von Introns, die in genomischen Sequenzen enthalten sind, welche mit den unter SEQ ID No. 1 angegebenen cDNA-Sequenzen korrespondieren. Die Isolierung und Identifizierung entsprechender Intron-Sequenzen kann beispielsweise unter Verwendung der unter SEQ ID No. 1 angegebenen Nukleinsäuremoleküle erfolgen, beispielsweise durch Durchmusterung einer genomischen DNA-Bibliothek.

Der Begriff "Hybridisierung" bedeutet im Rahmen dieser Erfindung eine Hybridisierung unter konventionellen Hybridisierungsbedingungen, vorzugsweise unter stringenten Bedingungen, wie sie beispielsweise in Sambrook et al., Molecular Cloning, A Laboratory Manual, 2. Aufl. (1989) Cold Spring Harbor Laboratory Press, Cold Spring Harbor, NY) beschrieben sind. Besonders bevorzugt bedeutet "Hybridisierung", daß eine Hybridisierung unter den folgenden Bedingungen auftritt:
- Hybridisierungspuffer. 2 x SSC;: 10 x Denhardt-Lösung (Fikoll 400 + PEG + BSA; Verhältnis 1:1:1); 0,1 % SDS; 5 mM EDTA; 50 mM Na₂HPO₄; 250 µg/ml Heringssperma-DNA; 50 µg/ml tRNA; oder
0,25 M Natriumphosphatpuffer pH 7,2;
1 mM EDTA
7% SDS
- Hybridisierungstemperatur: T = 65 bis 68°C
- Waschpuffer: 0,2 x SSC; 0,1 % SDS
- Waschtemperatur: T = 40 bis 68°C.
Nukleinsäuremoleküle, die mit den erfindungsgemäßen Nukleinsäuremolekülen hybridisieren, können prinzipiell aus jedem beliebigen Organismus (d.h. Prokaryonten oder Eukaryonten, insbesondere aus Bakterien, Pilzen, Algen, Pflanzen oder tierischen Organismen) stammen, der derartige Moleküle besitzt. Sie stammen vorzugsweise aus monokotylen oder dikotylen Pflanzen, insbesondere aus Nutzpflanzen und besonders bevorzugt aus stärkespeichernden Pflanzen, insbesondere aus Mais.
Nukleinsäuremoleküle, die mit den erfindungsgemäßen Molekülen hybridisieren, können z. B. aus genomischen oder aus cDNA-Bibliotheken verschiedener Organismen isoliert werden. Alternativ können sie durch gentechnische Methoden oder durch chemische Synthese hergestellt sein.

Die Identifizierung und Isolierung derartiger Nukleinsäuremoleküle aus Pflanzen oder anderen Organismen kann dabei unter Verwendung der erfindungsgemäßen Moleküle oder Teile dieser Moleküle bzw. der reversen Komplemente dieser Moleküle erfolgen, z. B. mittels Hybridisierung nach Standardverfahren (siehe z.B. Sambrook et al., 1989, Molecular Cloning, A Laboratory Manual, 2. Aufl. Cold Spring Harbor Laboratory Press, Cold Spring Harbor, NY).
Als Hybridisierungssonde können z. B. Nukleinsäuremoleküle verwendet werden, die exakt oder im wesentlichen die unter Seq ID No. 1 angegebene Nucleotidsequenz oder Teile dieser Sequenz aufweisen. Bei den als Hybridisierungssonde verwendeten Fragmenten kann es sich-auch um synthetische Fragmente handeln, die mit Hilfe der gängigen Synthesetechniken hergestellt wurden und deren Sequenz im wesentlichen mit der eines erfindungsgemäßen Nukleinsäuremoleküls übereinstimmt. Hat man Gene identifiziert und isoliert, die mit den erfindungsgemäßen Nukleinsäuresequenzen hybridisieren, ist eine Bestimmung der Sequenz und eine Analyse der Eigenschaften der von dieser Sequenz codierten Proteine erforderlich.

Ferner betrifft die Erfindung das Plasmid IR 65/87, das bei der Deutschen Sammlung für Mikroorganismen, Braunschweig, Deutschland unter der Nummer
DSM 12970 am 5. August 1999 hinterlegt worden ist, sowie das in der Insertion des Plasmids IR 65/87 enthaltene Nukleinsäuremolekül, das für ein Protein mit der enzymatischen Aktivität einer Stärkesynthase codiert. Darüber hinaus betrifft die vorliegende Erfindung Fragmente des in der Insertion des Plasmids IR 65/87 enthaltenen Nukleinsäuremoleküls, bevorzugt Fragmente, die die codierende Region oder einen Teil davon umfassen. Ferner betrifft die vorliegende Erfindung auch Nukleinsäuremoleküle, die mit dem in der Insertion des Plasmids IR 65/87 enthaltenem Nukleinsäuremolekül hybridisieren sowie Nukleinsäuremoleküle, die eine Sequenz aufweisen, die zu der gesamten oder einem Teil der Insertion des im Plasmid IR 65/87 enthaltenen Nukleinsäuremoleküls komplementär ist: Darüber hinaus betrifft die vorliegende Erfindung Nukleinsäuremoleküle, deren Nucleotidsequenz im Vergleich zu den Nukleinsäuremolekülen der Insertion des Plasmids IR 65/87 aufgrund der Degeneration des genetischen Codes abweicht.

Die vorliegende Erfindung betrifft auch Fragmente und allelischen Varianten der oben beschriebenen erfindungsgemäßen Nukleinsäuremoleküle.

Unter Fragmenten werden dabei Teile der erfindungsgemäßen Nukleinsäuremoleküle verstanden, die für ein erfindungsgemäßes Protein oder für Teile dieses Proteins codieren und die in der Regel Oligo- oder Polynucleotide sind, bestehend aus etwa 25 bis 150, bevorzugt aus mindestens 150, besonders bevorzugt mindestens 500, insbesondere mindestens 1000 und besonders bevorzugt aus mindestens 3500 Nucleotiden der erfindungsgemäßen Nukleinsäuremoleküle.
Unterdem Begriff "Fragment" wird in diesem Zusammenhang ein Teil der erfindungsgemäßen Nukleinsäuremoleküle verstanden, der für einen Teil des beschriebenen Proteins codiert und funktionell aktiv ist. Ferner kann das Fragment auch eine antisense-mRNA codieren oder in einem Molekül enthalten sein, das einen Cosuppressions- oder einen in-vivo-mutagenese-Effekt vermittelt. "Funktionell aktiv" bedeutet in diesem Zusammenhang, dass die biologische Aktivität des von dem erfindungsgemäßen Nukleinsäuremolekül codierten Proteins in einer erfindungsgemäßen Pflanzenzelle entweder erhöht oder erniedrigt wird.

Bei den allelischen Varianten kann es sich sowohl um natürlich auftretende Varianten handeln, als auch um synthetisch hergestellte oder durch rekombinante DNA-Techniken erzeugte Varianten.

Die Erfindung betrifft auch Derivate der oben beschriebenen erfindungsgemäßen Nukleinsäuremoleküle. Der Ausdruck "Derivat" bedeutet in diesem Zusammenhang, daß die Sequenzen dieser Moleküle sich von den Sequenzen der oben beschriebenen Nukleinsäuremoleküle an einer oder mehreren Positionen unterscheiden und einen hohen Grad an Homologie zu diesen Sequenzen, insbesondere zur codierenden Region der unter SEQ ID No. 1 angegebenen Nucleotidsequenz, aufweisen. Homologie bedeutet dabei eine Sequenzidentität von mindestens 50 %, insbesondere eine Identität von mindestens 70 %, vorzugsweise über 85 % und besonders bevorzugt über 95 %. Die Abweichungen zu den oben beschriebenen Nukleinsäuremolekülen können dabei durch Deletion, Substitution, Insertion oder Rekombination entstanden sein.

"Homologie" bedeutet im Rahmen der vorliegenden Erfindung, daß funktionelle und/oder strukturelle Äquivalenz zwischen den betreffenden Nukleinsäuremolekülen oder den, durch sie codierten, Proteinen besteht. Bei den Nukleinsäuremolekülen, die homolog zu den oben beschriebenen Molekülen sind und Derivate dieser Moleküle darstellen, handelt es sich in der Regel um Variationen dieser Moleküle, die Modifikationen darstellen, die dieselbe biologische Funktion ausüben. Es kann sich dabei sowohl um-natürlicherweise auftretende Variationen handeln, beispielsweise um Sequenzen aus anderen Organismen oder um Mutationen, wobei diese Mutationen auf natürliche Weise aufgetreten sein können oder durch gezielte Mutagenese eingeführt wurden.

Die von den verschiedenen Varianten (Fragmente, Derivate, allelische Varianten) der erfindungsgemäßen Nukleinsäuremoleküle codierten Proteine weisen bestimmte gemeinsame Charakteristika mit der unter Seq ID No. 2 definierten Aminosäuresequenz auf. Dazu können z.B. Enzymaktivität, Molekulargewicht, immunologische Reaktivität, Konformation etc. gehören, sowie physikalische Eigenschaften, wie z. B. das Laufverhalten in Gelelektrophoresen, chromatographisches Verhalten, Sedimentationskoeffizienten, Löslichkeit, spektroskopische Eigenschaften, Stabilität; pH-Optimum, Temperatur-Optimum etc..

Wichtige Charakteristika einer Stärkesynthase sind: i) ihre Lokalisation im Stroma der Plastiden pflanzlicher Zellen; ii) ihre Fähigkeit zur Synthese linearer α-1,4-verknüpfter Polyglucane unter Verwendung von ADP-Glucose als Substrat. Diese Aktivität kann bestimmt werden wie in Denyer und Smith (Planta 186 (1992), 609-617) beschrieben.
Die erfindungsgemäßen Nukleinsäuremoleküle können aus einem pro- oder eukaryontischen Organismus stammen, der die beschriebenen Gene exprimiert, vorzugsweise aus Pflanzen, insbesondere aus stärkesynthetisierenden bzw. stärkespeichernden Pflanzen. Diese können sowohl monokotyle oder auch dikotyle Pflanzen sein. Besonders bevorzugt sind dabei z. B. Getreidearten (wie Gerste, Roggen, Hafer, Weizen etc.), Mais, Reis, Erbse, Maniok, Kartoffel usw..
Bei den durch die erfindungsgemäßen Nukleinsäuremolekülen codierten Proteinen handelt es sich um eine bisher nicht identifizierte und charakterisierte Isoform einer pflanzlichen Stärkesynthase. Diese Proteine weisen die enzymatische Aktivität einer Stärkesynthase auf und zeigen im Bereich der Aminosäuren 740 bis 1170 der unter Seq ID No. 2 angegebenen Aminosäuresequenz eine signifikante Homologie zur SSIII aus Kartoffel (Marshall et al., Plant Cell 8, (1996), 1121-1135) und zu der als zSSlll bezeichneten Isoform (du1) aus Mais (Cao et al., Plant Physiol. 1.20, (1999), 205-215). Die durch die erfindungsgemäßen Nukleinsäuremoleküle codierten Proteine unterscheiden sich von der SSIII aus Kartoffel und von der zSSlll aus Mais signifikant durch ihren N-Terminus. Ferner unterscheidet sich der berechnete isoelektrische Punkt des unter SEQ ID No. 2 angegebenen Proteins signifikant von den berechneten isoelektrischen Punkten für die SSlll aus Kartoffel und für die zSSIII aus Mais. Darüber hinaus weist das unter SEQ ID No. 2 angegebene Protein im Vergleich zur zSSIII (berechnetes Molekulargewicht von ca. 188 kDa) ein deutlich verringertes berechnetes Molekulargewicht von ca. 132 kDa auf. lm Unterschied zur zSSlll werden die Gene der erfindungsgemäßen Isoform in jungen Blättern stärker exprimiert als im Endosperm.

Die vorliegende Erfindung betrifft daher in einer weiteren Ausführungsform die oben beschriebenen erfindungsgemäßen Nukleinsäuremoleküle, die Proteine mit der biologischen Aktivität einer Stärkesynthase codieren, wobei derartige Moleküle vorzugsweise Proteine codieren, die im Bereich des N-Terminus eine Homologie von mindestens 50%, bevorzugt von mindestens 65%, insbesondere von mindestens 80% und besonders bevorzugt von mindestens 95% zu der unter Seq ID No. 2 angegebenen Aminosäuresequenz aufweisen. Unter dem Begriff "N-Terminus" versteht man in diesem Zusammenhang die Aminosäuren 1 bis 150, bevorzugt die Aminosäuren 1 bis 300 und besonders bevorzugt die Aminosäuren 1 bis 480 der unter SEQ ID No. 2 angegebenen Aminosäuresequenz.

In einer weiteren Ausführungsform der Erfindung codieren die erfindungsgemäßen Nukleinsäuremoleküle Proteine mit der biologischen Aktiviät einer Stärkesynthase, wobei derartige Moleküle Proteine codieren, die einen berechneten isoelektrischen Punkt von pl = 6.95 pH ± 1.00 pH, vorzugsweise von pl = 6:95 pH ± 0.75 pH, besonders bevorzugt von pl = 6.95 pH ± 0.50 pH aufweisen.

In einer weiteren Ausführungsform codieren die erfindungsgemäßen Nukleinsäuremoleküle Proteine mit der biologischen Aktivität einer Stärkesynthase, die mindestens eine Deletion in mindestens einem der für Stärkesynthasen charakteristischen acht Sequenzmotive aufweisen, die von Cao et al. (Plant Physiol. 120, (1999), 205-215) beschrieben worden sind. Bevorzugt handelt es sich bei dem deletierten Motiv um das von Cao et al**.** (Plant Physiol. 120, (1999), 205-215) als Sequenzmotiv VII bezeichnete. Daher codieren in einer weiteren Ausführungsform die erfindungsgemäßen Nukleinsäuremoleküle für Proteine mit der biologischen Aktivität einer Stärkesynthase, die mindestens eine Deletion in einem oder mehreren der Sequenzmotive VII, ausgewählt aus der Gruppe bestehend aus
SHTIYAASDLFIIPSIFEPCGLTQMIAMRYGS (Seq ID No. 3);
SHLIYAGADFILVPSIFEPCGLTQLTAMRYGS (Seq ID No. 4);
SHLIYAGSDFILVPSIFEPCGLTQLVAMRYGT (Seq ID No. 5);
AHQMMAGADVLAVTSRFEPCGLIQLQGMRYGT (Seq ID No. 6);
AHQMMAGADVLAVTSRFEPCGLIQLQGMRYGT (Seq ID No. 7);
AHMITAGADFMLIPSRFEPCGLIQLHAMRYGT (Seq ID No. 8);
AHMITAG-ADFMLVPSRFEPCGLIQLHAMRYGT (Seq ID No. 9);
AHLIMAGADVLAVPSRFEPCGLIQLQGMRYGT (Seq ID No. 10);
AHKIIAGADFIVIPSRFEPCGLVQLHAMPYGT (Seq ID No. 11);
AHHIMAGADLLAVTSRFEPCGLIQLQGMRYGT (Seq ID No. 12);
AHHIMAGADVLAVTSRFEPCGLIQLQGMRYGT (Seq ID No. 13);
SHRITAGCDILLMPSRFEPCGLNQLYAMQYGT (Seq ID No. 14);
AHRITAGSDILLMPSRFEPCGLNQLYAMSYGT (Seq ID No. 15);
SHRITAGCDILLMPSRFEPCGLNQLYAMRYGT (Seq ID No. 16);
SHRITAGADILLMPSRFEPCALNQLYAMKYGT (Seq ID No. 17);
AHRITAGADIALMPSRFEPCGLNQLYAMAYGT (Seq ID No. 18);
SHRITAGCDILLMPSRFEPCGLNQLYAMQYGT (Seq ID No. 19);
SHRITAGCDILLMPSRFEPCGLNQLYAMQYGT (Seq ID No. 20);
AHRITAGADVLVMPSRFEPCGLNQLYAMAYGT (Seq ID No. 21);
AHRITAGADILLMPSRFEPCGLNQLYAMAYGT (Seq ID No. 22);
ARKLYASSDFILMPSYFEPCGLTQMIGMRYGC (Seq ID No. 23);
AHQIYAGSDMFLMPSKFEPCGLTQLYALRYGC (Seq ID No. 24);
AHQIYAGADLFLIPSLFEPCGLSQMIALRYGT (Seq ID No. 25);
AHQIYAGADLFLlPSLFEPCGLGQLIALQYGA (Seq ID. No. 26);
SHRIMGGADVILVPSRFEPCGLTQLYGSKYGT (Seq ID No. 27);
SHLMVAGGDVILVPSRFEPCGLTQLYGLQYGT (Seq ID No. 28) und
AHLIYGAADIIWPSNYEPCGLTQMIGLRYGA (Seq ID No. 29) (vgl. Sequenzmotiv VII wie in Cao et al., Plant Physiol. 120, (1999), S. 207, Tabelle 1 definiert) aufweisen.

Verfahren zur Identifizierung besagter Sequenzmotive sind dem Fachmann bekannt und können auf Aminosäuresequenzvergleichen mit den von Cao et al. beschriebenen charakteristischen Sequenzmotiven VII (s.o.) basieren.

In einer weiteren bevorzugten Ausführungsform der Erfindung codieren die erfindungsgemäßen Nukleinsäuremoleküle Proteine mit der Funktion einer Stärkesynthase, die eine Deletion von mindestens 2 Aminosäuren, besonders bevorzugt von mindestens 5 Aminosäuren, insbesondere von mindestens 10 Aminosäuren und besonders bevorzugt von mindestens 20 Aminosäuren in einem oder mehreren der Motive gemäß Seq ID No. 3 bis 29 aufweisen.

In einer weiteren besonders bevorzugten Ausführungsform der Erfindung weist besagtes Sequenzmotiv VII der durch die oben beschriebenen erfindungsgemäßen Nukleinsäuremoleküle codierten Proteine folgende Aminosäuresequenz auf: (1)SH--AMRYG-(11), wobei die Positionen 3, 4, 5 und 11 dieses Motivs von jeder beliebigen Aminosäure besetzt sein können. In Seq ID No. 2 beginnt das Sequenzmotiv VII bei Aminosäure 1067 (= S) und endet bei Aminosäure 1077 (=S). Besonders bevorzugt findet man an Position 3 eine Aminosäure ausgewählt aus der Gruppe bestehend aus T, L, M, Q oder R. An Position 4 findet man bevorzugt eine Aminosäure ausgewählt aus der Gruppe bestehend aus 1, L oder M. An Position 5 findet man bevorzugt eine Aminosäure ausgewählt aus der Gruppe bestehend aus Y, M, T oder V. An Position 11 findet man bevorzugt eine Aminosäure ausgewählt aus der Gruppe bestehend aus S, T, C oder A.

Die vorliegende Erfindung betrifft auch die Verwendung der oben beschriebenen erfindungsgemäßen Nukleinsäuremoleküle zur Durchmusterung von Nukleinsäurebibliotheken, insbesondere von cDNA und genomischen Bibliotheken und/oder als Hybridisierungssonde. Darüber hinaus betrifft die vorliegende Erfindung auch die Verwendung der oben beschriebenen erfindungsgemäßen Nukleinsäuremoleküle zur Herstellung transgener Pflanzenzellen oder transgener Pflanzen.

Ferner betrifft die Erfindung Vektoren, insbesondere Plasmide, Cosmide, Viren, Bakteriophagen und andere in der Gentechnik gängige Vektoren, die die oben beschriebenen erfindungsgemäßen Nukleinsäuremoleküle enthalten.

In einer bevorzugten Ausführungsform sind die in den Vektoren enthaltenen Nukleinsäuremoleküle verknüpft mit regulatorischen Elementen, die die Transkription und Synthese einer translatierbaren RNA in prokaryontischen oder eukaryontischen Zellen oder im zellfreien System gewährleisten. Regulatorische Elemente zur Expression der erfindungsgemäßen Nukleinsäuremoleküle in Mikroorganismen (z. B. *E*. *coli, S*. *cerevisiae*) sind ausreichend in der Literatur beschrieben. Promotoren, die eine besonders starke Expression des nachgeschalteten Gens erlauben, sind z. B. der T7-Promotor (Studier et al., Methods in Enzymology 185, (1990), 60-89), lacuv5, trp, trp-lacUV5 (DeBoer et al., in: Rodriguez and Chamberlin (Eds), Promotors, Structure and Function; Praeger, New York, (1982), 462-481; DeBoer et al, Proc. Natl. Acad. Sci. USA (1983), 21-25), lp1, rac (Boros et al., Gene 42 (1986), 97-100). In der Regel erreichen die Proteinmengen von der Mitte bis gegen Ende der logarithmischen Phase des Wachstumszyklus der Mikroorganismen ihren Höhepunkt. Zur Synthese von Proteinen werden daher bevorzugt induzierbare Promotoren verwendet. Diese führen oft zu höheren Ausbeuten an Protein als konstitutive Promotoren. Die Verwendung starker konstitutiver Promotoren führt über die ständige Transkription und Translation eines clonierten Gens oft dazu, daß Energie für andere wesentliche Zellfunktionen verlorengeht und dadurch das Zellwachstum verlangsamt wird (Bemard R. Glick/ Jack J. Pasternak. Molekulare Biotechnologie (1995), Spektrum Akademischer Verlag GmbH, Heidelberg Berlin Oxford, S. 342.). Um ein Optimum an Proteinmenge zu erreichen, wird daher oft ein zweistufiges Verfahren angewendet. Zuerst werden die Wirtszellen unter optimalen Bedingungen bis zu einer relativ hohen Zelldichte kultiviert. Im zweiten Schritt wird dann die Transkription je nach Art des eingesetzten Promotors induziert. Besonders geeignet ist in diesem Zusammenhang ein durch Lactose- oder IPTG (=Isopropyl-β-D-thiogalactopyranosid) induzierbarer tac-Promotor (deBoer et al., Proc. Natl. Acad. Sci. USA 80 (1983), 21-25). Terminationssignale für die Transkription sind ebenfalls in der Literatur beschrieben.
Regulatorische Elemente zur Expression der erfindungsgemäßen Nukleinsäuremoleküle in pflanzlichen Zellen werden im Zusammenhang mit den erfindungsgemäßen Pflanzenzellen beschrieben.
Die Expression der erfindungsgemäßen Nukleinsäuremoleküle in prokaryontischen Zellen, beispielsweise in *Escherichia coli,* ist insofern wichtig, als daß auf diese Weise eine.genauere Charakterisierung der enzymatischen Aktivitäten der Enzyme, für die diese Moleküle codieren, ermöglicht wird. Es ist insbesondere möglich, das Produkt, das von den entsprechenden Enzymen synthetisiert wird, in Abwesenheit anderer, in der pflanzlichen Zelle an der Stärkesynthese beteiligter Enzyme, zu charakterisieren. Dies läßt Rückschlüsse auf die Funktion zu, die das entsprechende Protein bei der Stärkesynthese in der Pflanzenzelle ausübt.
Darüber hinaus ist es möglich, mittels gängiger molekularbiologischer Techniken (siehe z. B. Sambrook et al., 1989, Molecular Cloning, A Laboratory Manual, 2. Auf). Cold Spring Harbor Laboratory Press, Cold Spring Harbor, NY) verschiedenartige Mutationen in die erfindungsgemäßen Nukleinsäuremoleküle einzuführen, wodurch es zur Synthese von Proteinen mit eventuell veränderten biologischen Eigenschaften kommt. Hierbei ist zum einen die Erzeugung von Detetionsmutanten möglich, bei denen durch fortschreitende Deletionen vom 5'- oder vom 3'- Ende der codierenden DNA-Sequenz Nukleinsäuremoleküle erzeugt werden, die zur Synthese entsprechend verkürzter Proteine führen. Durch derartige Deletionen am 5'-Ende der Nucleotidsequenz ist es beispielsweise möglich, Aminosäuresequenzen zu identifizieren, die für die Translokation des Enzyms in die Plastiden verantwortlich sind (Transitpeptide). Dies erlaubt es, gezielt Enzyme herzustellen, die durch Entfernen der entsprechenden Sequenzen nicht mehr in den Plastiden, sondern im Cytosol lokalisiert sind oder aufgrund der Addition von anderen Signalsequenzen in anderen Kompartimenten lokalisiert sind.
Auch der Austausch des eigenen Transitpeptids gegen ein anderes, die plastidäre Lokalisation vermittelndes Transitpeptid ist denkbar. Als plastidäre Signalsequenz kann beispielsweise die der Ferrodoxin:NADP⁺-Oxidoreductase (FNR) aus Spinat verwendet werden. Diese Sequenz enthält den 5' nichttranslatierten Bereich sowie die flankierende Transitpeptidsequenz der cDNA des plastidären Proteins Ferrodoxin:NADP⁺-Oxidoreductase aus Spinat (Nukleotid -171 bis + 165; Jansen et al., Current Genetics 13, (1988), 517-522).
Ferner kann als plastidäre Signalsequenz beispielsweise das Transitpeptid des waxy-Proteins aus Mais plus die ersten 34 Aminosäuren des maturen waxy-Proteins (Klösgen et al., Mol Gen Genet. 217, (1989), 155-161) verwendet werden. Darüber hinaus kann das Transitpeptid des waxy-Proteins aus Mais (s. o.) auch ohne die ersten 34 Aminosäuren des maturen waxy-Proteins verwendet werden.

Andererseits ist auch die Einführung von Punktmutationen in die erfindungsgemäßen Nukleinsäuremoleküle denkbar an Positionen, bei denen eine Veränderung der Aminosäuresequenz einen Einfluß beispielsweise auf die Enzymaktivität oder die Regulierung des Enzyms hat. Auf diese Weise können z. B. Mutanten hergestellt werden, die einen veränderten k_{cat} und/oder Kₘ-Wert besitzen oder nicht mehr den normalerweise in der Zelle vorliegenden Regulationsmechanismen über allosterische Regulation oder kovalente Modifizierung unterliegen.
Des weiteren können Mutanten hergestellt werden, die eine veränderte Substrat- oder Produktspezifität aufweisen, wie z. B. Mutanten, die als Substrat ADP-Glucose-6-Phosphat anstatt ADP-Glucose verwenden. Weiterhin können Mutanten hergestellt werden, die ein verändertes Aktivitäts-Temperatur-Profil aufweisen.
Für die gentechnische Manipulation in prokaryontischen Zellen können die erfindungsgemäßen Nukleinsäuremoleküle oder Teile dieser Moleküle in Plasmide eingebracht werden, die eine Mutagenese oder eine Sequenzveränderung durch Rekombination von DNA-Sequenzen erlauben. Mit Hilfe von Standardverfahren (vgl. Sambrook et al., 1989, Molecular Cloning: A laboratory manual, 2. Aufl., Cold Spring Harbor Laboratory Press, NY, USA) können Basenaustausche vorgenommen oder natürliche oder synthetische Sequenzen hinzugefügt werden. Für die Verbindung der DNA-Fragmente untereinander können an die Fragmente Adaptoren oder Linker angesetzt werden. Ferner können Manipulationen, die passende Restriktionsschnittstellen zur Verfügung stellen oder die überflüssige DNA oder Restriktionsschnittstellen entfernen, eingesetzt werden. Wo Insertionen, Deletionen oder Substitutionen in Frage kommen, können in vitro-Mutagenese, "primer repair", Restriktion oder Ligation verwendet werden. Als Analysemethode werden im allgemeinen eine Sequenzanalyse, eine Restriktionsanalyse und weitere biochemisch-molekularbiologische Methoden durchgeführt.

Ferner betrifft die vorliegende Erfindung Vektoren, die die oben beschriebenen erfindungsgemäßen Nukleinsäuremoleküle enthalten, wobei die Nukleinsäuremoleküle in sense-Orientierung mit regulatorischen Elementen verknüpft sind, die die Transkription und Synthese einer translatierbaren RNA in pro- oder eukaryontischen Zellen gewährleisten.
Die Bedeutung des Begriffs "sense-Orientierung" ist dem Fachmann bekannt.

In einer weiteren Ausführungsform betrifft die Erfindung Wirtszellen, insbesondere prokaryontische oder eukaryontische Zellen, die mit einem oben beschriebenen erfindungsgemäßen Nukleinsäuremolekül oder einem erfindungsgemäßen Vektor transformiert sind, sowie Zellen, die von derart transformierten Zellen abstammen und ein erfindungsgemäßes Nukleinsäuremolekül oder einen erfindungsgemäßen Vektor enthalten. Dabei handelt es sich vorzugsweise um bakterielle Zellen, besonders bevorzugt um pflanzliche Zellen.

Ferner betrifft die vorliegende Erfindung ein Verfahren zur Herstellung einer Wirtszelle wobei eine Wirtszelle genetisch modifiziert wird durch die Einführung eines erfindungsgemäßen Nukleinsäuremoleküls und/oder eines erfindungsgemäßen Vektors. Die Wirtszelle kann dabei sowohl prokaryontischen als auch eukaryontischen Ursprungs sein. Dabei handelt es sich vorzugsweise um bakterielle Zellen, besonders bevorzugt um pflanzliche Zeiten.

Der Begriff "genetisch modifiziert" bedeutet dabei im Zusammenhang mit der vorliegenden Erfindung, daß die Wirtszelle, insbesondere eine pflanzliche Zelle, durch Einführung eines erfindungsgemäßen Nukleinsäuremoleküls in ihrer genetischen Information verändert ist, und daß das Vorhandensein oder die Expression des erfindungsgemäßen Nukleinsäuremoleküls zu einer phänotypischen Veränderung führt. Phänotypische Veränderung bedeutet dabei vorzugsweise eine meßbare Veränderung einer oder mehrerer Funktionen der Zellen. Beispielsweise zeigen genetisch modifizierte erfindungsgemäße Pflanzenzellen eine Verringerung der Aktivität des erfindungsgemäßen Proteins oder eine Erhöhung der Aktivität des erfindungsgemäßen Proteins.

Gegenstand der Erfindung sind ferner die Proteine oder biologisch aktive Fragmente davon, die durch die erfindungsgemäßen Nukleinsäuremoleküle codiert werden, sowie Verfahren zu deren Herstellung, wobei eine erfindungsgemäße Wirtszelle unter Bedingungen kultiviert wird, die die Synthese des Proteins erlauben, und anschließend das Protein aus den kultivierten Zellen und/oder dem Kulturmedium isoliert wird. Die charakteristischen Eigenschaften der erfindungsgemäßen Proteine wurden bereits oben im Zusammenhang mit der Beschreibung der korrespondierenden erfindungsgemäßen Nukleinsäuremoleküle beschrieben.

Durch die Bereitstellung der erfindungsgemäßen Nukleinsäuremoleküle ist es nun möglich, mit Hilfe gentechnischer Methoden in den Stärkemetabolismus von Pflanzen einzugreifen, wie es bisher nicht möglich war, und ihn dahingehend zu verändern, daß es zur Synthese einer modifizierten Stärke kommt, die beispielsweise in ihren physikalisch-chemischen Eigenschaften, insbesondere dem Amylose/Amylopektin-Verhältnis, dem Verzweigungsgrad, der durchschnittlichen Kettenlänge, dem Phosphatgehalt, dem Verkleisterungsverhalten, der .
Stärkekorngröße und/oder der Stärkekomform (Stärkekommorphologie) im Vergleich zu in Wildtyppflanzen synthetisierter Stärke verändert ist. Durch eine Erhöhung der Aktivität der erfindungsgemäßen Proteine beispielsweise durch Überexpression entsprechender Nukleinsäuremoleküle, oder durch die Bereitstellung von Mutanten, die nicht mehr den zelleigenen Regulationsmechanismen unterliegen und/oder unterschiedliche Temperaturabhängigkeiten in bezug auf ihre Aktivität besitzen, besteht die Möglichkeit der Ertragssteigerung in entsprechend gentechnisch veränderten Pflanzen.
Die wirtschaftliche Bedeutung der Möglichkeit des Eingriffs in die Stärkesynthese allein bei Mais ist offensichtlich: Mais ist weltweit die wichtigste Pflanze zur Stärkegewinnung. Ca. 80% der weltweit jährlich produzierten Stärke wird aus Mais gewonnen.
Möglich ist somit die Expression der erfindungsgemäßen Nukleinsäuremoleküle in pflanzlichen Zellen, um die Aktivität der entsprechenden Stärkesynthase zu erhöhen. Ferner ist es möglich, die erfindungsgemäßen Nukleinsäuremoleküle nach dem Fachmann bekannten Methoden zu modifizieren, um erfindungsgemäß Stärkesynthasen zu erhalten, die nicht mehr den zelleigenen Regulationsmechanismen unterliegen, bzw. veränderte Temperaturabhängigkeiten oder Substrat- bzw. Produktspezifitäten aufweisen.
Bei der Expression der erfindungsgemäßen Nukleinsäuremoleküle in Pflanzen besteht grundsätzlich die Möglichkeit, daß das synthetisierte Protein in jedem beliebigen Kompartiment der pflanzlichen Zelle lokalisiert sein kann. Um die Lokalisation in einem bestimmten Kompartiment zu erreichen, muß die die Lokalisation in Plastiden gewährleistende Sequenz deletiert werden und die verbleibende codierende Region gegebenenfalls mit DNA-Sequenzen verknüpft werden, die die Lokalisierung in dem jeweiligen Kompartiment gewährleisten.
Derartige Sequenzen sind bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Beispiele für plastidäre Signalsequenzen wurden bereits oben in anderem Zusammenhang genannt.

Die vorliegende Erfindung betrifft somit auch transgene Pflanzenzellen, die mit einem erfindungsgemäßen Nukleinsäuremolekül oder einem erfindungsgemäßen Vektor transformiert wurden, sowie transgene Pflanzenzellen, die von derartig tranformierten Zellen abstammen. Derartige Zellen enthalten ein erfindungsgemäßes Nukleinsäuremolekül, wobei dieses vorzugsweise mit regulatorischen DNA-Elementen verknüpft ist, die die Transkription in pflanzlichen Zellen gewährleisten, insbesondere mit einem Promotor.
Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Transformation von Protoplasten mittels Polyethylenglykol, die Injektion, die Elektroporation von DNA, die Einbringung der DNA mittels des biolistischen Ansatzes sowie weitere Möglichkeiten.

Die Verwendung der Agrobakterien-vermittelten Transformation von Pflanzenzellen ist intensiv untersucht und ausreichend in EP-A2-0120516; Hoekema, IN: The Binary Plant Vector System Offsetdrukkerij Kanters B.V., Alblasserdam (1985), Chapter V; Fraley et al., Crit. Rev. Plant Sci. 4, 1-46 und An et al. EMBO. J. 4, (1985), 277-287 beschrieben worden. Für die Transformation von Kartoffel, siehe z. B. Rocha-Sosa et al. (EMBO J. 8, (1989), 29-33.).
Auch die Transformation monokotyler Pflanzen mittels Agrobakterium basierender Vektoren wurde beschrieben (Chan et al., Plant Mol. Biol. 22, (1993), 491-506; Hiei et al., Plant J. 6, (1994) 271-282; Deng et al, Science in China 33, (1990), 28-34; Wilmink et al., Plant Cell Reports 11, (1992), 76-80; May et al., Bio/Technology.13, (1995), 486-492; Conner und Domisse, Int. J. Plant Sci. 153 (1992), 550-555; Ritchie et al, Transgenic Res. 2, (1993), 252-265). Ein alternatives System zur Transformation von monokotylen Pflanzen ist die Transformation mittels des biolistischen Ansatzes (Wan und Lemaux, Plant Physiol. 104, (1994), 37-48; Vasil et al., BiolTechnology 11. (1993), 1553-1558; Ritala et al., Plant Mol. Biol. 24, (1994), 317-325; Spencer et al., Theor. Appl. Genet. 79, (1990), 625-631), die Protoplastentransformation, die Elektroporation von partiell permeabilisierten Zellen, die Einbringung von DNA mittels Glasfasern. Insbesondere die Transformation von Mais wurde in der Literatur mehrfach beschrieben (vgl. z. B. WO 9506128A2, EP-A2- 0513849, EP-A1-0465875, EP-A1-292435; Fromm et al., Biotechnology 8, (1990), 833-844; Gordon-Kamm et al., Plant Cell 2, (1990), 603-618; Koziel et al., Biotechnology 11 (1993), 194-200; Moroc et al., Theor. Appl. Genet. 80, (1990), 721-726).
Auch die erfolgreiche Transformation anderer Getreidearten wurde bereits beschrieben, z. B. für Gerste (Wan und Lemaux, s.o.; Ritala et al., s.o.; Krens et al., Nature 296, (1982), 72-74) und für Weizen (Nehra et al.; Plant J. 5, (1994), 285-297).
Regulatorische Elemente für die Expression der erfindungsgemäßen Nukleinsäuremoleküle in Pflanzenzellen sind prinzipiell jeder in pflanzlichen Zellen aktive Promotor, Enhancer, Terminator etc.. Der Promotor kann dabei so gewählt sein, daß die Expression in den erfindungsgemäßen Pflanzen konstitutiv erfolgt oder nur in einem bestimmten Gewebe, zu einem bestimmten Zeitpunkt der Pflanzenentwicklung oder zu einem durch äußere Einflüsse determinierten Zeitpunkt. In Bezug auf die Pflanze kann der Promotor homolog oder heterolog sein. Geeignete Promotoren sind z. B. der Promotor der 35S RNA des Cauliflower Mosaic Virus (siehe beispielsweise US5352605) und der Ubiquitin-Promotor (siehe beispielsweise US5614399) für eine konstitutive Expression, der Patatingen-Promotor B33 (Rocha-Sosa et al., EMBO J. 8 (1989), 23-29) für eine knollenspezifische Expression in Kartoffeln oder ein Promotor, der eine Expression lediglich in photosynthetisch aktiven Geweben sicherstellt, z.B. der ST-LS1-Promotor (Stockhaus et al., Proc. Natl. Acad. Sci. USA 84 (1987), 7943-7947; Stockhaus et al., EMBO J. 8 (1989), 2445-2451), der Ca/b-Promotor (s. beispielsweise US 5656496, US 5639952, Bansal et al., Proc. Natl. Acad. Sci.USA 89, (1992), 3654-3658) und der Rubisco SSU-Promotor (s. beispielsweise US 5034322, US 4962028). Es können jedoch auch Promotoren verwendet werden, die nur zu einem durch äußere Einflüsse determinierten Zeitpunkt aktiviert werden (siehe beispielsweise WO9307279A1). Von besonderem Interesse können hierbei Promotoren von heat-shock Proteinen sein, die eine einfache Induktion erlauben.
Darüber hinaus gewährleisten samenspezifische Promotoren, wie z. B. der USP-Promoter aus Vicia faba, eine samenspezifische Expression in Vicia faba und anderen Pflanzen (Fiedler et al., Plant Mol. Biol. 22, (1993), 669-679; Bäumlein et al., Mol. Gen. Genet. 225, (1991), 459-467). Ferner können auch fruchtspezifische Promotoren eingesetzt werden, wie z.B. beschrieben in der WO9101373A1.
Besonders bevorzugt werden Promotoren für eine endosperm-spezifische Expression verwendet, wie z. B. der Glutelinpromotor (Leisy et al., Plant Mol. Biol. 14, (1990), 41-50; Zheng et al., Plant J. 4, (1993), 357-366), der HMG-Promotor aus Weizen, der USP-Promotor, der Phaseolinpromotor oder Promotoren von Zein-Genen aus Mais (Pedersen et al., Cell 29, (1982), 1015-1026; Quatroccio et al., Plant Mol. Biol. 15 (1990), 81-93). Mit Hilfe endospermspezifischer Promotoren ist es möglich, die Transkriptmenge der erfindungsgemäßen Nukleinsäuremoleküle im Endosperm im Vergleich zum Endosperm entsprechender Wildtyppflanzen zu erhöhen.

Besonders bevorzugt wird der shrunken-1-Promotor (sh-1) aus Mais (Werr et al., EMBO J. 4, (1985), 1373-1380) verwendet.
Ferner kann eine Terminationssequenz vorhanden sein, die der korrekten Beendigung der Transkription dient sowie der Addition eines Poly-A-Schwanzes an das Transkript, dem eine Funktion bei der Stabilisierung der Transkripte beigemessen wird. Derartige Elemente sind in der Literatur beschrieben (vgl. z. B. Gielen et al., EMBO J. 8 (1989), 23-29) und sind beliebig austauschbar.

Darüber hinaus ist es möglich, mit Hilfe der erfindungsgemäßen Nukleinsäuremoleküle Pflanzenzellen und Pflanzen zu erzeugen, bei denen die Aktivität eines erfindungsgemäßen Proteins verringert ist. Dies führt zur Synthese einer Stärke mit veränderten chemischen und/oder physikalischen Eigenschaften verglichen mit Stärke aus Wildtyppflanzenzellen.
Ein weiterer Gegenstand der Erfindung sind somit auch transgene Pflanzenzellen, in denen die Aktivität eines erfindungsgemäßen Proteins verringert ist im Vergleich zu entsprechenden nicht genetisch modifizierten Pflanzenzellen von Wildtyppflanzen.

Der Begriff "Wildtyppflanze"/"Wildtyppflanzenzelle" bedeutet im Zusammenhang mit der vorliegenden Erfindung, daß es sich um Pflanzen/Pflanzenzellen handelt, die als Ausgangsmaterial für die Herstellung der erfindungsgemäßen Pflanzen/Pflanzenzellen dienten, d.h. deren genetische Information, abgesehen von der eingeführten genetischen Modifikation, der einer erfindungsgemäßen Pflanze/Pflanzenzelle entspricht.

Die Herstellung von Pflanzenzellen mit einer verringerten Aktivität eines erfindungsgemäßen Proteins kann beispielsweise erzielt werden durch die Expression einer entsprechenden antisense-RNA, oder die Expression eines entsprechend konstruierten Ribozyms, das spezifisch Transkripte spaltet, die eines der erfindungsgemäßen Proteine codieren: Ferner kann die Verringerung der Aktivität durch die Einführung von solchen DNA-Molekülen erreicht werden, die über einen Cosuppressionseffekt zu einer Verringerung der Expression von endogenen Genen führen, die ein erfindungsgemäßes Protein codieren. Des weiteren kann die Herstellung von Pflanzenzellen mit einer verringerten Aktivität eines erfindungsgemäßen Proteins mittels in vivo-Mutagenese erfolgen. Dabei können über homologe Rekombination Mutationen oder Insertionen in ein das erfindungsgemäße Protein codierende endogene Gen eingeführt werden. Die Mutation oder Insertion führt zu einer Verringerung der Expression des endogenen Gens, das ein erfindungsgemäßes Protein codiert, oder zur Synthese eines inaktiven erfindungsgemäßen Proteins.

Vorzugsweise wird zur Reduzierung der Aktivität eines erfindungsgemäßen Proteins, in pflanzlichen Zellen eine antisense-RNA exprimiert.
Hierzu können zum einen DNA-Moleküle verwendet werden, die die gesamte für ein erfindungsgemäßes Protein codierende Sequenz einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Ferner können aber auch genomische DNA-Moleküle verwendet werden, die beispielsweise für ein Intron codieren. Es können im allgemeinen Sequenzen bis zu einer Mindestlänge von 15 bp, vorzugsweise einer Länge von 100-500 bp, für eine effiziente antisense-Inhibition insbesondere Sequenzen mit einer Länge über 500 bp verwendet werden: In der Regel werden DNA-Moleküle verwendet, die kürzer als 5000 bp, vorzugsweise Sequenzen, die kürzer als 2500 bp sind. Bevorzugt werden DNA-Moleküle verwendet, die homolog in Bezug auf die zu transformierende Pflanzenspezies sind.
Möglich ist auch die Verwendung von DNA-Sequenzen, die einen hohen Grad an Homologie zu den Sequenzen der erfindungsgemäßen DNA-Moleküle aufweisen, aber nicht vollkommen identisch sind. Die minimale Homologie sollte größer als ca. 65 % sein. Die Verwendung von Sequenzen mit Homologien zwischen 95 und 100 % ist zu bevorzugen.

Alternativ kann die Verringerung der Aktivität des erfindungsgemäßen Proteins in den Pflanzenzellen auch durch einen Cosuppressionseffekt erfolgen. Das Verfahren ist dem Fachmann bekannt und ist beispielsweise beschrieben in Jorgensen (Trends Biotechnol. 8 (1990), 340-344), Niebel et al., (Curr. Top. Microbiol. Immunol. 197 (1995), 91-103), Flavell et al. (Curr. Top. Microbiol. lmmunol. 197 (1995), 43-46), Palaqui und Vaucheret (Plant. Mol. Biol. 29 (1995), 149'159), Vaucheret et al., (Mol. Gen. Genet. 248 (1995), 311-317), de Borne et al. (Mol. Gen. Genet. 243 (1994), 613-621) und anderen Quellen.
Wie im Falle der oben beschriebenen aritisense-Technologie können auch hier sowohl DNA-Moleküle verwendet werden, die die gesamte codierende Region des erfindungsgemäßen Nukleinsäuremoleküls umfassen als auch DNA-Moleküle, die nur Teile der codierenden Sequenz umfassen. Ebenfalls ist die Verwendung von Introns denkbar.

Die Expression von Ribozymen zur Verringerung der Aktivität von bestimmten Enzymen in Zellen ist dem Fachmann bekannt und ist beispielsweise beschrieben in EP-B1-0321201. Die Expression von Ribozymen in pflanzlichen Zellen wurde z.B. beschrieben in Feyter et al. (Mol. Gen. Genet. 250, (1996), 329-338).

Ferner kann die Verringerung der Aktivität des erfindungsgemäßen Nukleinsäuremoleküls in den Pflanzenzellen auch durch die sogenannte "in vivo-Mutagenese" erreicht werden, bei der durch Transformation von Zellen ein hybrides RNA-DNA-Oligonucleotid ("Chimeroplast") in Zellen eingeführt wird (Kipp, P.B. et al., Poster Session beim "5^{th} International Congress of Plant Molecular Biology, 21.-27. September 1997, Singapore; R. A. Dixon und C.J. Arntzen, Meeting report zu "Metabolic Engineering in Transgenic Plants", Keystone Symposia, Copper Mountain, CO, USA, TIBTECH 15, (1997), 441-447; internationale Patentanmeldung WO 9515972A1; Kren et al., Hepatology 25, (1997), 1462-1468; Cole-Strauss et al., Science 273, (1996), 1386-1389).
Ein Teil der DNA-Komponente des RNA-DNA-Oligonucleotids ist homolog zu einer Nukleinsäuresequenz eines endogenen für ein erfindungsgemäßes Nukleinsäuremolekül codierenden Gens, weist jedoch im Vergleich zur Nukleinsäuresequenz des endogenen Gens eine Mutation auf oder enthält eine heterologe Region, die von den homologen Regionen umschlossen ist.
Durch Basenpaarung der homologen Regionen des RNA-DNA-Oligonucleotids und des endogenen Nukleinsäuremoleküls, gefolgt von homologer Rekombination kann die in der DNA-Komponente des RNA-DNA-Oligonucleotids enthaltene Mutation oder heterologe Region in das endogene Gen einer Pflanzenzelle übertragen werden. Dies führt zu einer Verringerung der Aktivität eines erfindungsgemäßen Proteins.

Ferner ist dem Fachmann bekannt, daß er die Aktivität eines erfindungsgemäßen Proteins durch die Expression von nicht-funktionellen Derivaten insbesondere transdominanten Mutanten solcher Proteine und/oder durch die Expression von Antagonisten/Inhibitoren solcher Proteine erreichen kann. Antagoisten/Inhibitoren solcher Proteine umfassen beispielsweise Antikörper, Antikörperfragmente oder Moleküle mit ähnlichen Bindungseigenschaften. Beispielsweise wurde ein cytoplasmatischer scFv Antikörper eingesetzt, um die Aktivität des Phytochrom A Proteins in gentechnisch veränderten Tabakpflanzen zu modulieren (Owen, Bio/Technology 10, (1992), 790-794; Review: Franken, E., Teuschel, U. und Hain, R., Current Opinion in Biotechnology 8, (1997), 411-416; Whitelam, Trends Plant Sci. 1, (1996), 268-272).

Der Begriff "Verringerung der Aktivität" bedeutet im Rahmen der vorliegenden Erfindung eine Verringerung der Expression endogener Gene, die ein erfindungsgemäßes Protein codieren und/oder eine Verringerung der Menge an erfindungsgemäßem Protein in den Zellen, insbesondere eine Verringerung der enzymatischen Aktivität des erfindungsgemäßen Proteins in den Zellen.
Die Verringerung der Expression kann beispielsweise bestimmt werden durch Messung der Menge an das erfindungsgemäße Protein codierenden Transkripten, z. B. durch Northem-Blot-Analyse. Eine Verringerung bedeutet dabei vorzugsweise eine Verringerung der Menge an Transkripten im Vergleich zu entsprechenden nicht genetisch modifizierten Zellen um mindestens 50%, bevorzugt um mindestens 70%, besonders bevorzugt um mindestens 85% und ganz besonders bevorzugt um mindestens 95%.
Die Verringerung der Menge an erfindungsgemäßem Protein kann beispielsweise bestimmt werden durch Western-Blot-Analyse. Eine Verringerung bedeutet dabei vorzugsweise eine Verringerung der Menge an erfindungsgemäßem Protein im Vergleich zu entsprechenden nicht genetisch modifizierten Zellen um mindestens 50%, bevorzugt um mindestens 70%, besonders bevorzugt um mindestens 85% und ganz besonders bevorzugt um mindestens 95%.
Die Verringerung der enzymatischen Aktivität des erfindungsgemäßen Proteins kann beispielsweise bestimmt werden wie bei Denyer und Smith (Planta 186 (1992), 609-617) beschrieben. Eine Verringerung der enzymatischen Aktiviät im Vergleich zu entsprechenden nicht genetisch modifizierten-Zellen bedeutet dabei vorzugsweise eine Verringerung um mindestens 50%, bevorzugt um mindestens 70%, besonders bevorzugt um mindestens 85% und ganz besonders bevorzugt um mindestens 95%.

Für den Begriff "genetisch modifiziert" gilt dasselbe, was bereits oben in anderem Zusammenhang gesagt wurde.

Gegenstand der vorliegenden Erfindung sind daher insbesondere auch transgene Pflanzenzellen,
a) die mindestens ein DNA-Molekül enthalten, das zur Synthese mindestens einer antisense-RNA führen kann, welche eine Verringerung der Expression von endogenen Genen, die ein erfindungsgemäßes Protein codieren, bewirkt;
b) die mindestens ein DNA-Molekül enthalten, das über einen Cosuppressionseffekt zu einer Verringerung der Expression von endogenen Genen, die ein erfindungsgemäßes Protein codieren, führt;
c) die mindestens ein DNA-Molekül enthalten, das zur Synthese mindestens eines Ribozyms führt, welches spezifisch Transkripte von endogenen Genen, die ein erfindungsgemäßes Protein codieren, spaltt; und/oder
d) die aufgrund einer in-vivo-Mutagenese eine Mutation oder eine Insertion einer heterologen DNA-Sequenz in mindestens einem endogenen, ein erfindungsgemäßes Protein codierendem Gen aufweisen, wobei die Mutation oder Insertion eine Verringerung der Expression des Gens oder die Synthese eines inaktiven erfindungsgemäßen Proteins bewirkt.

Ferner ist es möglich, mit Hilfe der erfindungsgemäßen Nukleinsäuremoleküle Pflanzenzellen und Pflanzen zu erzeugen, bei denen die Aktivität eines erfindungsgemäßen Proteins erhöht ist. Dies führt zur Synthese einer Stärke mit veränderten chemischen und/oder physikalischen Eigenschaften verglichen mit Stärke aus Wildtyppflanzenzellen und Wildtyppflanzen.
Ein weiterer Gegenstand der Erfindung sind auch transgene Pflanzenzellen, in denen die Aktivität eines erfindungsgemäßen Proteins erhöht ist im Vergleich zu entsprechenden nicht genetisch modifizierten Pflanzenzellen von Wildtyppflanzen.

Zur Erzeugung von erfindungsgemäßen Pflanzenzellen, die eine erhöhte Aktivität des erfindungsgemäßen Proteins im Vergleich zu entsprechenden nicht genetisch modifizierten Wildtyppflanzenzellen aufweisen, werden erfindungsgemäße Nukleinsäuremoleküle in sense-Orientierung verwendet, die die codierende Region einer erfindungsgemäßen Stärkesynthase umfassen. In einer weiteren Ausführungsform können auch Teile der codierenden Region verwendet werden, sofern sie für ein katalytisch aktives Stärkesynthaseprotein codieren. In einer besonders bevorzugten Ausführungsform werden die unter Seq ID No. 1. angegebenen Nukleirisäuremoleküle verwendet.

Der Begriff "Erhöhung der Aktivität" bedeutet dabei im Rahmen der vorliegenden Erfindung eine Erhöhung der Expression endogener Gene, die ein erfindungsgemäßes Protein codieren und/oder eine Erhöhung der Menge an erfindungsgemäßem Protein in den Zellen, insbesondere eine Erhöhung der enzymatischen Aktivität des erfindungsgemäßen Proteins in den Zellen.

Die Erhöhung der Expression kann beispielsweise bestimmt werden durch Messung der Menge an das erfindungsgemäße Protein codierenden Transkripten, z.B. durch Northem-Blot-Analyse. Eine Erhöhung bedeutet dabei vorzugsweise eine Erhöhung der Menge an Transkripten im Vergleich zu entsprechenden nicht genetisch modifizierten Zellen um mindestens 50%, bevorzugt um mindestens 100%, insbesondere um mindestens 500% und ganz besonders bevorzugt um mindestens 1500%.
Die Erhöhung der Menge an erfindungsgemäßem Protein kann beispielsweise' bestimmt werden durch Western-Blot-Analyse. Eine Erhöhung bedeutet dabei vorzugsweise eine Erhöhung der Menge an erfindungsgemäßem Protein im Vergleich zu entsprechenden nicht genetisch modifizierten Zellen um mindestens 50%, bevorzugt um mindestens 100%, insbesondere um mindestens 500% und besonders bevorzugt um mindestens 1500%.
Besonders bevorzugt kann die Erhöhung der enzymatischen Aktivität des erfindungsgemäßen Proteins bestimmt werden wie bei Denyer und Smith (s.o.) beschrieben.
Eine Erhöhung der enzymatischen Aktiviät im Vergleich zu entsprechenden nicht genetisch modifizierten Zellen bedeutet dabei vorzugsweise eine Erhöhung um mindestens 50%, bevorzugt um mindestens 100%, insbesondere um mindestens 500% und besonders bevorzugt um mindestens 1500%.

In einer bevorzugten Ausführungsform der Erfindung handelt es sich bei den erfindungsgemäßen transgenen Pflanzenzellen mit erhöhter Aktivität eines erfindungsgemäßen Proteins im Vergleich zu Pflanzenzellen aus Wildtyppflanzen um solche Pflanzenzellen, die aus stärkespeichernden Geweben stammen, bevorzugt aus Knollen und dem Endosperm, insbesondere aus dem Endosperm von Maispflanzen.

Es wurde überraschenderweise gefunden, daß Pflanzenzellen, bei denen die Aktivität des erfindungsgemäßen Proteins, insbesondere in Pflanzenzellen des Endosperms, im Vergleich zu entsprechenden Wildtyppflanzen erhöht ist, eine Stärke synthetisieren, die in ihren physikalisch-chemischen Eigenschaften im Vergleich zu in Wildtyppflanzen, insbesondere im Endosperm, synthetisierter Stärke verändert ist, so daß diese für spezielle Verwendungszwecke besser geeignet ist.

Die erfindungsgemäßen Pflanzenzellen können zu jeder beliebigen Pflanzenspezies gehören, d.h. sowohl zu monokotyledonen als auch zu dikotyledonen Pflanzen. Bevorzugt handelt es sich um Pflanzenzellen aus landwirtschaftlichen Nutzpflanzen, d.h. aus Pflanzen, die vom Menschen kultiviert werden für Zwecke der Ernährung oder für technische, insbesondere industrielle Zwecke. Vorzugsweise betrifft die Erfindung faserbildende (z. B. Flachs, Hanf, Baumwolle), ölspeichernde (z. B. Raps; Sonnenblume, Sojabohne), zuckerspeichemde (z. B. Zuckerrübe, Zuckerrohr, Zuckerhirse, Banane) und proteinspeichemde Pflanzen (z. B. Leguminosen).. In einer weiteren bevorzugten Ausführungsform betrifft die Erfindung Pflanzenzellen aus Futterpflanzen (z. B. Futter- und Weidegräser, Alfalfa, Klee etc.), Gemüsepflanzen (z. B. Tomate, Salat, Chicoree).
In einer besonders bevorzugten Ausführungsform betrifft die Erfindung Pflanzenzellen aus stärkespeichernden Pflanzen (z.B. Weizen, Gerste, Hafer, Roggen, Kartoffel, Mais, Reis, Erbse, Maniok, Mungbohne); insbesondere bevorzugt sind Pflanzenzellen aus Mais, Reis, Weizen und Kartoffel.

Die erfindungsgemäßen Pflanzenzellen können zur Regeneration ganzer Pflanzen verwendet werden.
Die durch Regeneration der-erfindungsgemäßen transgenen Pflanzenzellen erhältlichen Pflanzen sind ebenfalls Gegenstand der vorliegenden Erfindung.

Ferner sind Gegenstand der Erfindung Pflanzen, die die oben beschriebenen erfindungsgemäßen Pflanzenzellen enthalten. Bei den erfindungsgemäßen Pflanzen kann es sich prinzipiell um Pflanzen jeder beliebigen Pflanzenspezies handeln, d.h. sowohl monokotyle als auch dikotyle Pflanzen. Bevorzugt handelt es sich um Nutzpflanzen, d.h. Pflanzen, die vom Menschen kultiviert werden für Zwecke der Ernährung oder für technische, insbesondere industrielle Zwecke. Vorzugsweise betrifft die Erfindung Pflanzenzellen aus faserbildenden (z. B. Flachs, Hanf, Baumwolle), ölspeichemden (z. B. Raps, Sonnenblume, Sojabohne), zuckerspeichemden (z. B. Zuckerrübe, Zuckerrohr, Zuckerhirse, Banane) und proteinspeichemden Pflanzen (z. B. Leguminosen) In einer weiteren bevorzugten Ausführungsform betrifft die Erfindung Futterpflanzen (z. B. Futter- und Weidegräser (Alfalfa, Klee etc.)), Gemüsepflanzen (z.B. Tomate, Salat, Chicoree).
In einer besonders bevorzugten Ausführungsform betrifft die Erfindung stärkespeichernde Pflanzen (z. B. Weizen, Gerste, Hafer, Roggen, Kartoffel, Mais, Reis, Erbse, Maniok, Mungbohne), insbesondere bevorzugt sind Mais-, Reis-, Weizen- und Kartoffelpflanzen.

In einer weiteren bevorzugten Ausführungsform weisen die erfindungsgemäßen Pflanzen eine erhöhte Aktivität eines erfindungsgemäßen Proteins in Pflanzenzellen stärkespeichernder Gewebe auf im Vergleich zu entsprechenden nicht genetisch modifizierten Pflanzenzellen von Wildtyppflanzen, bevorzugt in Pflanzenzellen aus Knollen oder des Endosperms, besonders bevorzugt in Pflanzenzellen des Endosperms von Maispflanzen.

In einer weiteren Ausführungsform der Erfindung weisen Pflanzen, die die erfindungsgemäßen Pflanzenzellen mit erhöhter Aktivität des erfindungsgemäßen Proteins enthalten, im Vergleich zu entsprechenden nicht modifizierten Wildtyppflanzen einen erhöhten Ertrag und/oder einen erhöhten Stärkegehalt auf.

Der Begriff "erhöhter Ertrag" bedeutet dabei vorzugsweise eine Erhöhung der Produktion an Inhaltsstoffen, insbesondere an Stärke, und/oder Biomasse, insbesondere, wenn diese am Frischgewicht pro Pflanze gemessen wird.
Der Begriff "erhöhter Stärkegehalt" bedeutet dabei, daß der Gehalt an Stärke in erfindungsgemäßen Pflanzenzellen um mindestens 10%, bevorzugt um mindestens 20%, insbesondere um mindestens 30% und besonders bevorzugt um mindestens 40% im Vergleich zu Pflanzenzellen von nicht modifizierten Wildtyp-Pflanzen erhöht ist.
Verfahren zur Bestimmung des Stärkegehalts sind dem Fachmann bekannt.
Eine derartige Erhöhung des Ertrags und/oder des Stärkegehalts bezieht sich vorzugsweise auf emtebare Pflanzenteile wie Samen, Früchte, Speicherwurzeln, Knollen, Wurzeln, Blüten, Knospen, Sprosse, Stämme oder Holz.
Die Erhöhung des Ertrags beträgt erfindungsgemäß mindestens 3%, bezogen auf die Biomasse und/oder Inhaltsstoffe, im Vergleich zu entsprechenden nicht-transformierten Pflanzen desselben Genotyps, wenn diese unter denselben Bedingungen kultiviert werden, bevorzugt mindestens 5%, insbesondere mindestens. 10% und besonders bevorzugt mindestens 20% oder gar 40% im Vergleich zu Wildtyppflanzen.

Die vorliegende Erfindung betrifft auch Verfahren zur Herstellung transgener Pflanzen, wobei
a) eine pflanzliche Zelle genetisch modifiziert wird durch die Einführung eines erfindungsgemäßen Nukleinsäuremoleküls und/oder eines erfindungsgemäßen Vektors; und
b) aus der Zelle eine Pflanze regeneriert wird; und-gegebenenfalls
c) aus der Pflanze nach- b) weitere Pflanzen erzeugt werden.

Für die laut Schritt a) eingeführte genetische Modifikation gilt dasselbe, was bereits oben in anderem Zusammenhang erläutert wurde. Beispielsweise zeigen genetisch modifizierte Pflanzenzellen eine Verringerung der Aktivität des erfindungsgemäßen Proteins oder eine Erhöhung der Aktivität des erfindungsgemäßen Proteins. Die Regeneration von Pflanzen gemäß Schritt b) kann nach dem Fachmann bekannten Methoden erfolgen.
Die Erzeugung weiterer Pflanzen gemäß Schritt c) der erfindungsgemäßen Verfahren kann z.B. erfolgen durch vegetative Vermehrung (beispielsweise über Stecklinge, Knollen oder über Calluskultur und Regeneration ganzer Pflanzen) oder durch generative Vermehrung. Die generative Vermehrung findet dabei vorzugsweise kontrolliert statt, d.h. es werden ausgewählte Pflanzen mit bestimmten Eigenschaften miteinander gekreuzt und vermehrt.

Die vorliegende Erfindung betrifft auch die durch die erfindungsgemäßen Verfahren erhältlichen Pflanzen.

Die vorliegende Erfindung betrifft auch Vermehrungsmaterial erfindungsgemäßer Pflanzen sowie der gemäß den erfindungsgemäßen Verfahren hergestellten transgenen Pflanzen. Der Begriff Vermehrungsmaterial umfaßt dabei jene Bestandteile der Pflanze, die geeignet sind zur Erzeugung von Nachkommen auf vegetativem oder generativem Weg. Für die vegetative Vermehrung eignen sich beispielsweise Stecklinge, Calluskulturen, Rhizome oder Knollen. Anderes Vermehrungsmaterial umfaßt beispielsweise Früchte, Samen, Sämlinge, Protoplasten, Zellkulturen etc.. Vorzugsweise handelt es sich bei dem Vermehrungsmaterial um Knollen und Samen.

In einer weiteren Ausführungsform betrifft die vorliegende Erfindung emtbare .. Pflanzenteile erfindungsgemäßer Pflanzen, wie Früchte, Speicherwurzeln, Wurzeln, Blüten, Knospen, Sprosse oder Stämme, vorzugsweise Samen oder Knollen; sowie Futtermittel, die diese erntebaren Pflanzenteile, bevorzugt Samen oder Knollen, enthalten. Erfindungsgemäße emtebare Pflanzenteile, bevorzugt Samen oder Knollen und/oder Futtermittel können durch einen veränderten Energiewert, bevorzugt einen erhöhten Energiewert, gekennzeichnet sein.

Unter dem Begriff "Energiewert" soll in diesem Zusammenhang insbesondere die "verdauliche Energie" verstanden werden. Der Begriff "verdauliche Energie" ist definiert als: verdauliche Energie = Bruttoenergie des Futters minus Kotbrennwert (Landwirtschaftliches Lehrbuch 2: Tierzucht, Ed.:D. Schmidt, 5. Auflage, 1982, Eugen Ulmer GmbH & Co, S. 244). Unter der Bruttoenergie ist der in Joule meßbare Gesamtbrennwert eines Futtermittels zu verstehen. Verfahren zur Bestimmung des "Energiewerts" bzw. der "verdaulichen Energie" sind dem Fachmann bekannt. Die Erhöhung des Energiewerts beträgt erfindungsgemäß mindestens 3%, bevorzugt mindestens 10%, insbesondere mindestens 30% und besonders bevorzugt mindestens 60%.

Die erfindungsgemäßen transgenen Pflanzenzellen und Pflanzen synthetisieren aufgrund der Expression bzw. zusätzlichen Expression eines erfindungsgemäßen Nukleinsäuremoleküls und/oder aufgrund der Erhöhung oder der Verringerung der Aktivität eines erfindungsgemäßen Proteins eine Stärke, die beispielsweise in ihren physikalisch-chemischen Eigenschaften, insbesondere dem Amylose/Amylopektin-Verhältnis, dem Verzweigungsgrad, der durchschnittlichen Kettenlänge, dem Phosphatgehalt, dem Verkleisterungsverhalten, der Stärkekomgröße und/oder der Stärkekornform im Vergleich zu in Wildtyppflanzen synthetisierter Stärke verändert ist. Insbesondere kann eine solche Stärke im Hinblick auf die Viskosität und/oder die Gelbildungseigenschaften von Kleistern dieser Stärke im Vergleich zu Wildtypstärke verändert sein.
Gegenstand der vorliegenden Erfindung ist somit auch die aus den erfindungsgemäßen transgenen Pflanzenzellen und/oder den erfindungsgemäßen Pflanzen und/oder dem erfindungsgemäßen Vermehrungsmaterial erhältliche Stärke.

Aufgrund der veränderten chemisch-physikalischen Eigenschaften zeigen die erfindungsgemäßen Stärken veränderte funktionelle Eigenschaften. Wichtige funktionelle Eigenschaften der Stärke bzw. ihrer wäßrigen Lösungen sind beispielsweise das Retrogradierungsverhalten, die Filmbildungseigenschaften, die Gelfestigkeit, die Viskosität, die Farbstabilität, die enzymatische Verdaubarkeit, die Gefrier-Tau-Stabitität, die Säurestabilität, die Scherstabilität, die Transparenz, das Wasserbindevermögen, die Verkleisterungseigenschaften sowie Binde-und Klebeeigenschaften. Die erfindungsgemäßen Stärken können nach dem Fachmann bekannten Verfahren modifiziert werden und eignen sich in unmodifizierter oder modifizierter Form für verschiedene Verwendungen im Nahrungsmittel- oder Nicht-Nahrungsmittel-Bereich.

Grundsätzlich läßt sich die Einsatzmöglichkeit der Stärke in zwei große Bereiche unterteilen. Der eine Bereich umfaßt die Hydrolyseprodukte der Stärke, hauptsächlich Glucose und Glucanbausteine, die über enzymatische oder chemische Verfahren erhalten werden. Sie dienen als Ausgangsstoff für weitere chemische Modifikationen und Prozesse, wie Fermentation. Für eine Reduktion der Kosten kann hierbei die Einfachheit und kostengünstige Ausführung eines Hydrölyseverfahrens von Bedeutung sein. Gegenwärtig verläuft es im wesentlichen enzymatisch unter Verwendung, von Amyloglucosidase. Vorstellbar wäre eine Kosteneinsparung durch einen geringeren Einsatz von Enzymen. Eine Strukturveränderung der Stärke, z. B. Oberflächenvergrößerung des Körns, leichtere Verdaulichkeit durch geringeren Verzweigungsgrad oder eine sterische Struktur, die die Zugänglichkeit für die eingesetzten Enzyme begrenzt, könnte dies bewirken.
Der andere Bereich, in dem die Stärke wegen ihrer polymeren Struktur als sogenannte native Stärke verwendet wird, gliedert sich in zwei weitere Einsatzgebiete:

### 1. Nahrungsmittelindustrie

Stärke ist ein klassischer Zusatzstoff für viele Nahrungsmittel, bei denen sie im wesentlichen die Funktion des Bindens von wäßrigen Zusatzstoffen übernimmt bzw. eine Erhöhung der Viskosität oder aber eine erhöhte Gelbildung hervorruft. Wichtige Eigenschaftsmerkmale sind das Fließ- und Sorptionsverhalten, die Quell-und Verkleisterungstemperatur, die Viskosität und Dickungsleistung, die Löslichkeit der Stärke, die Transparenz und Kleisterstruktur, die Hitze-, Scher- und Säurestabilität, die Neigung zur Retrogradation, die Fähigkeit zur Filmbildung, die Gefrier/Taustabilität, die' Verdaulichkeit sowie die Fähigkeit zur Komplexbildung mit z. B. anorganischen oder organischen Ionen.

### 2. Nicht-Nahrungmittelindustrie

In diesem großen Bereich kann die Stärke als Hilfsstoff für unterschiedliche Herstellungsprozesse bzw. als Zusatzstoff in technischen Produkten eingesetzt. Bei der Verwendung der Stärke als Hilfsstoff ist hier insbesondere die Papier- und Pappeindustrie zu nennen. Die Stärke dient dabei in erster Linie zur Retardation (Zurückhaltung von Feststoffen), der Abbindung von Füllstoff- und Feinstoffteilchen, als Festigungsstoff und zur Entwässerung. Darüber hinaus werden die günstigen Eigenschaften der Stärke in bezug auf die Steifigkeit, die Härte, den Klang, den Griff, den Glanz, die Glätte, die Spaltfestigkeit sowie die Oberflächen ausgenutzt.

### 2.1 Papier- und Pappeindustrie

Innerhalb des Papierherstellungsprozesses sind vier Anwendungsbereiche, nämlich Oberfläche, Strich, Masse und Sprühen, zu unterscheiden. Die Anforderungen an die Stärke in bezug auf die Oberflächenbehandlung sind im wesentlichen ein hoher Weißegrad, eine angepaßte Viskosität, eine hohe Viskositätsstabilität, eine gute Filmbildung sowie eine geringe Staubbildung. Bei der Verwendung im Strich spielt der Feststoffgehalt, eine angepaßte, Viskosität, ein hohes Bindevermögen sowie eine hohe Pigmentaffinität eine wichtige Rolle. Als Zusatz zur Masse ist eine rasche, gleichmäßige, verlustfreie Verteilung, eine hohe mechanische Stabilität und eine vollständige Zurückhaltung im Papiervlies von Bedeutung. Beim Einsatz der Stärke im Sprühbereich sind ebenfalls ein angepaßter Feststoffgehalt, hohe Viskosität sowie ein hohes Bindevermögen von Bedeutung.

### 2.2 Klebstoffindustrie

Ein großer Einsatzbereich der Stärken besteht in der Klebstoffindustrie; wo man die Einsatzmöglichkeiten in vier Teilbereiche gliedert: die Verwendung als reinem Stärkeleim, die Verwendung bei mit speziellen Chemikalien aufbereiteten Stärkeleimen, die Verwendung von Stärke als Zusatz zu synthetischen Harzen und Polymerdispersionen sowie die Verwendung von Stärken als Streckmittel für synthetische Klebstoffe. 90 % der Klebstoffe auf Stärkebasis werden in den Bereichen Wellpappenherstellung, Herstellung von Papiersäcken, Beuteln und Tüten, Herstellung von Verbundmaterialien für Papier und Aluminium, Herstellung von Kartonagen und Wiederbefeuchtungsleim für Briefumschläge, Briefmarken usw. eingesetzt.

### 2.3 Textil- und Textilpflegemittelindustrie

Ein großes Einsatzfeld für die Stärken als Hilfsmittel und Zusatzstoff ist der Bereich Herstellung von Textilien und Textitpflegemitteln. Innerhalb der Textilindustrie sind die folgenden vier Einsatzbereiche zu unterscheiden: Der Einsatz der Stärke als Schlichtmittel, d.h. als Hilfsstoff zur Glättung und Stärkung des Klettverhaltens zum Schutz gegen die beim Weben angreifenden Zugkräfte sowie zur Erhöhung der Abriebfestigkeit beim Weben, Stärke als Mittel zur Textilaufrüstung vor allem nach qualitätsverschlechternden Vorbehandlungen, wie Bleichen, Färben usw., Stärke als Verdickungsmittel bei der Herstellung von Farbpasten zur Verhinderung von Farbstoffdiffusionen sowie Stärke als Zusatz zu Kettungsmitteln für Nähgarne.

### 2.4 Baustoffindustrie

Der vierte Einsatzbereich ist die Verwendung der Stärken als Zusatz bei Baustoffen. Ein Beispiel ist die Herstellung von Gipskartonplatten, bei der die im Gipsbrei vermischte Stärke mit dem Wasser verkleistert, an die Oberfläche der Gipsplatte diffundiert und dort den Karton an die Platte bindet. Weitere Einsatzbereiche sind die Beimischung zu Putz- und Mineralfasern. Bei Transportbeton werden Stärkeprodukte-zur Verzögerung der Abbindung eingesetzt.

### 2.5 Bodenstabilisation

Ein weiterer Markt für die Stärke bietet sich bei der Herstellung von Mitteln zur Bodenstabilisation an, die bei künstlichen Erdbewegungen zum temporären Schutz der Bodenpartikel gegenüber Wasser eingesetzt werden. Kombinationsprodukte aus der Stärke und Polymeremulsionen sind nach heutiger Kenntnis in ihrer Erosions- und verkrustungsmindernden Wirkung den bisher eingesetzten Produkten gleichzusetzen, liegen preislich aber deutlich unter diesen.

### 2.6 Einsatz bei Pflanzenschutz- und Düngemitteln

Ein Einsatzbereich liegt bei der Verwendung der Stärke in Pflanzenschutzmitteln zur Veränderung der spezifischen Eigenschaften der Präparate. So kann die Stärke zur Verbesserung der Benetzung von Pflanzenschutz- und Düngemitteln, zur dosierten Freigabe der Wirkstoffe, zur Umwandlung flüssiger, flüchtiger und/oder übelriechender Wirkstoffe in mikrokristalline, stabile, formbare Substanzen, zur Mischung inkompatibler Verbindungen und zur Verlängerung der Wirkdauer durch Verminderung der Zersetzung eingesetzt werden.

### 2.7 Pharmaka, Medizin und Kosmetikindustrie

Ein weiteres Einsatzgebiet besteht im Bereich der Pharmaka, Medizin und Kosmetikindustrie. In der pharmazeutischen Industrie kann die Stärke als Bindemittel für Tabletten oder zur Bindemittelverdünnung in Kapseln eingesetzt werden. Weiterhin kann die Stärke als Tablettensprengmittel dienen, da sie nach dem Schlucken Flüssigkeit absorbieren und nach kurzer Zeit soweit quellen, daß der Wirkstoff freigesetzt wird. Medizinische Gleit- und' Wundpuder basieren aus qualitativen Gründen auf Stärke. Im Bereich der Kosmetik werden Stärken beispielsweise als Träger von Puderzusatzstoffen, wie Düften und Salicylsäure eingesetzt. Ein relativ großer Anwendungsbereich für die Stärke liegt bei Zahnpasta.

### 2.8 Stärkezusatz zu Kohlen und Briketts

Einen Einsatzbereich bietet die Stärke als Zusatzstoff zu Kohle und Brikett. Kohle kann mit einem Stärkezusatz quantitativ hochwertig agglomeriert bzw. brikettiert werden, wodurch ein frühzeitiges Zerfallen der Briketts verhindert wird. Der Stärkezusatz liegt bei Grillkohle zwischen 4 und 6 %, bei kalorierter Kohle zwischen 0,1 und 0,5 %. Des weiteren gewinnen Stärken als Bindemittel an Bedeutung, da durch ihren Zusatz zu Kohle und Brikett der Ausstoß schädlicher Stoffe deutlich vermindert werden kann.

### 2.9 Erz- und Kohleschlammaufbereitung

Die Stärke kann ferner bei der Erz- und Kohleschlammaufbereitung als Flockungsmittel eingesetzt werden.

### 2.10 Gießereihilfsstoff

Ein weiterer Einsatzbereich besteht als Zusatz zu Gießereihilfsstoffen. Bei verschiedenen Gußverfahren werden Kerne benötigt, die aus Bindemittelversetzten Sänden hergestellt werden. Als Bindemittel wird heute überwiegend Bentonit eingesetzt, das mit modifizierten Stärken, meist Quellstärken, versetzt ist.
Zweck des Stärkezusatzes ist die Erhöhung der Fließfestigkeit sowie die Verbesserung der Bindefestigkeit. Darüber hinaus können die Quellstärken weitere produktionstechnische Anforderungen, wie im kalten Wasser dispergierbar, rehydratisierbar, gut in Sand mischbar und hohes Wasserbindungsvermögen, aufweisen.

### 2.11 Einsatz in der Kautschukindustrie

In der Kautschukindustrie kann die Stärke zur Verbesserung der technischen und optischen Qualität eingesetzt werden. Gründe sind dabei die Verbesserung des Oberflächenglanzes, die Verbesserung des Griffs und des Aussehens, dafür wird Stärke vor der Kaltvulkanisation auf die klebrigen gummierten Flächen von Kautschukstoffen gestreut, sowie die Verbesserung der Bedruckbarkeit des Kautschuks.

### 2.12 Herstellung von Lederersatzstoffen

Eine weitere Absatzmöglichkeit der modifizierten Stärken besteht bei der Herstellung von Lederersatzstoffen.

### 2.13 Stärke in synthetischen Polymeren

Auf dem Kunststoffsektor zeichnen sich folgende Einsatzgebiete ab: die Einbindung von Stärkefolgeprodukten in den Verarbeitungsprozeß (Stärke ist nur Füllstoff, es besteht keine direkte Bindung zwischen synthetischem Polymer und Stärke) oder alternativ die Einbindung von Stärkefolgeprodukten in die Herstellung von Polymeren (Stärke und Polymer gehen eine feste Bindung ein).

Die Verwendung der Stärke als reinem Füllstoff ist verglichen mit den anderen Stoffen wie Talkum nicht wettbewerbsfähig. Anders sieht es aus, wenn die spezifischen Stärkeeigenschaften zum Tragen kommen und hierdurch das Eigenschaftsprofil der Endprodukte deutlich verändert wird. Ein Beispiel hierfür ist die Anwendung von Stärkeprodukten bei der Verarbeitung von Thermoplasten, wie Polyäthylen. Hierbei werden die Stärke und das synthetische Polymer durch Koexpression im Verhältnis von 1 : 1 zu einem 'master batch' kombiniert, aus dem mit granuliertem Polyäthylen unter Anwendung herkömmlicher Verfahrenstechniken diverse Produkte hergestellt werden. Durch die Einbindung von Stärke in Polyäthylenfolien kann eine erhöhte Stoffdurchlässigkeit bei Hohlkörpern, eine verbesserte Wasserdampfdurchlässigkeit, ein verbessertes Antistatikverhalten, ein verbessertes Antiblockvefialten sowie eine verbesserte Bedruckbarkeit mit wäßrigen Farben erreicht werden.
Eine andere Möglichkeit ist die Anwendung der Stärke in Polyurethanschäumen. Mit der Adaption der Stärkederivate sowie durch die verfahrenstechnische Optimierung ist es möglich, die-Reaktion zwischen synthetischen Polymeren und den Hydroxygruppen der Stärken gezielt zu steuern. Das Ergebnis sind Polyurethanfolien, die durch die Anwendung von Stärke folgende Eigenschaftsprofile erhalten: eine Verringerung des Wärmeausdehnungskoeffizienten, Verringerung des Schrumpfverhaltens, Verbesserung des Druck/Spannungsverhaltens, Zunahme der Wasserdampfdurchlässigkeit ohne Veränderung der Wasseraufnahme, Verringerung der Entflammbarkeit und der Aufrißdichte, kein Abtropfen brennbarer Teile, Halogenfreiheit und verminderte Alterung Nachteile, die gegenwärtig noch vorhanden sind, sind verringerte Druckfestigkeit sowie eine verringerte Schtagfestigkeit.
Die Produktentwicklung beschränkt sich inzwischen nicht mehr nur auf Folien. Auch feste Kunststoffprodukte, wie Töpfe, Platten und Schalen, sind mit einem Stärkegehalt von über 50 % herzustellen. Des weiteren sind Stärke/Polymermischungen günstig zu beurteilen, da sie eine sehr viel höhere biologische Abbaubarkeit aufweisen.
Außerordentliche Bedeutung haben weiterhin auf Grund ihres extremen Wasserbindungsvermögen Stärkepfropfpolymerisate gewonnen. Dies sind Produkte mit einem Rückgrat aus Stärke und einer nach dem Prinzip des Radikalkettenmechanismus aufgepfropften Seitengitters eines synthetischen Monomers. Die heute verfügbaren Stärkepfropfpolymerisate zeichnen sich durch ein besseres Binde- und Rückhaltevermögen von bis zu 1000 g Wasser pro g Stärke bei hoher Viskosität aus. Die Anwendungsbereiche für diese Superabsorber haben sich in den letzten Jahren stark ausgeweitet und liegen im Hygienebereich mit Produkten wie Windeln und Unterlagen sowie im landwirtschaftlichen Sektor, z. B. bei Saatgutpillierungen.

Entscheidend für den Einsatz der neuen, gentechnisch veränderten Stärken sind zum einen die Struktur, Wassergehalt, Proteingehalt, Lipidgehalt, Fasergehalt, Asche/Phosphatgehalt, Amylose/Amylopektinverhältnis, Molmassenverteilung, Verzweigungsgrad, Korngröße und -form sowie Kristallinität, zum anderen auch die Eigenschaften, die in folgende Merkmale münden: Fließ- und Sorptionsverhalten, Verkleisterungstemperatur, Viskosität, Dickungsleistung, Löslichkeit, Kleisterstruktur und -transparenz, Hitze-, Scher- und Säurestabilität, Retrogradationsneigung, Gelbildung, Gefrier/Taustabilität, Komplexbildung, Jodbindung, Filmbildung, Klebekraft, Enzymstabilität, Verdaulichkeit und Reaktivität.
Die Erzeugung modifizierter Stärken mittels gentechnischer Eingriffe in einer transgenen Pflanze kann zum einen die Eigenschaften der aus der Pflanze gewonnenen Stärke dahingehend verändern, daß weitere Modifikationen mittels chemischer oder physikalischer Verfahren nicht mehr notwendig erscheinen. Zum anderen können die durch gentechnische Verfahren veränderten Stärken weiteren chemischen und/oder physikalischen Modifikationen unterworfen werden, was zu weiteren Verbesserungen der Qualität für bestimmte der oben beschriebenen Einsatzgebiete führt. Diese chemischen und physikalischen Modifikationen sind grundsätzlich bekannt. Insbesondere handelt es sich dabei um Modifikationen durch:
- Hitzebehandlung,
- Säurebehandlung,
- Erzeugung von Stärkeethern
   Stärke-Alkylether, O-Allylether, Hydroxylalkylether, O-Carboxylmethylether, N-haltige Stärkeether, P-haltige Stärkeether, S-haltige Stärkeether
- Erzeugung von vernetzten Stärken
- Erzeugung von Stärke-Pfropf-Polymerisaten
- Oxidation und
- Veresterungen, welche zur Entstehung von Phosphat-, Nitrat-, Sulfat-, Xanthat-, Acetat- und Citratstärken führen. Weitere organische Säuren können ebenfalls zur Veresterung eingesetzt werden.

Die vorliegende Erfindung betrifft ferner ein Verfahren zur Herstellung einer modifizierten Stärke umfassend den Schritt der Extraktion der Stärke aus einer oben beschriebenen erfindungsgemäßen Pflanze(nzelle) und/oder aus stärkespeichernden Teilen einer solchen Pflanze. Vorzugsweise umfaßt ein solches Verfahren auch den Schritt des Emtens der kultivierten Pflanzen und/oder stärkespeichernder Teile dieser Pflanzen vor der Extraktion der Stärke und besonders bevorzugt ferner den Schritt der Kultivierung erfindungsgemäßer Pflanzen vor dem Ernten. Verfahren zur Extraktion der Stärke von Pflanzen oder von stärkespeichernden Teilen von Pflanzen sind dem Fachmann bekannt. Weiterhin sind Verfahren zur Extraktion der Stärke aus verschiedenen stärkespeichernden Pflanzen beschrieben, z. B. in "Starch: Chemistry and Technology (Hrsg.: Whistler, BeMiller und Paschall (1994), 2. Ausgabe, Academic Press Inc. London Ltd; ISBN 0-12-746270-8; siehe.z. B. Kapitel XII, Seite 412-468: Mais und Sorghum-Stärken: Herstellung; von Watson; Kapitel XIII; Seite 469-479: Tapioca-, Arrowroot- und Sagostärken: Herstellung; von Corbishley und Miller; Kapitel XIV, Seite 479-490: Kartoffelstärke: Herstellung und Verwendungen; von Mitch; Kapitel XV, Seite 491 bis 506: Weizenstärke: Herstellung, Modifizierung und Verwendungen; von Knight und Oson; und Kapitel XVI, Seite 507 bis 528: Reisstärke: Herstellung und Verwendungen; von Rohmer und Klem; Maisstärke: Eckhoff et al., Cereal Chem. 73 (1996) 54-57, die Extraktion von Maisstärke im industriellen Maßstab wird in der Regel durch das sogenannte "wet milling" erreicht.)). Vorrichtungen, die für gewöhnlich bei Verfahren zur Extraktion von Stärke von Pflanzenmaterial verwendet werden, sind Separatoren, Dekanter, Hydrocyclone, Sprühtrockner und Wirbelschichttrockner.

Gegenstand der vorliegenden Erfindung ist ferner Stärke, die durch das oben beschriebene erfindungsgemäße Verfahren erhältlich ist.

Die Erfindung betrifft ferner Stärke erhältlich aus den erfindungsgemäßen Pflanzenzellen und/oder erfindungsgemäßen Pflanzen sowie Stärke erhältlich aus stärkespeichernden Geweben, insbesondere Knollen und Körner, erfindungsgemäßer Pflanzen.

In einer weiteren Ausführungsform betrifft die vorliegende Erfindung die Verwendung der erfindungsgemäßen Stärken, im industriellen Bereich, vorzugsweise zur Herstellung von Nahrungsmitteln, Futtermitteln und Papier.

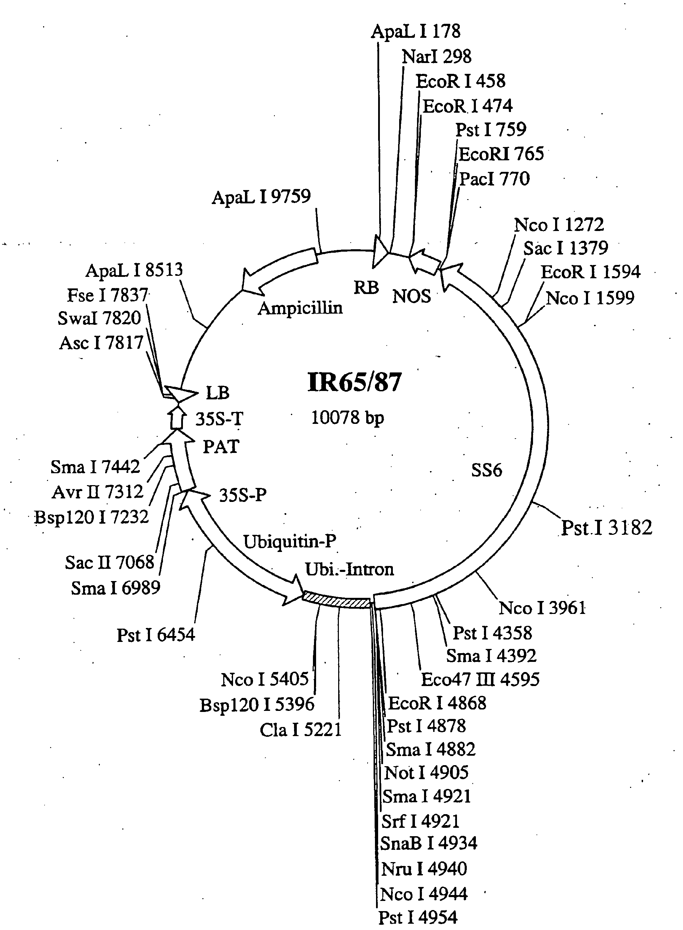

Fig. 1: Plasmidkarte IR 65/87
   LB: T-DNA left border
   35S-T: 35S-Terminator (Frank et al., Cell 21, (1980), 285-294)
   PAT: Phosphinothricin-Resistenz, EP-A2-0275957
   35S-P: CaMV 35 S Promotor (Frank et al., Cell 21, (1980), 285-294)
   Ubiquitin-P: Ubiquitin-Promotor (Christensen et al., Plant Mol. Biol. 18, (1992), 675-689)
   Ubi.-Intron: Ubiquitin-Intron (Christensen et al., Plant Mol. Biol. 18, (1992), 675-689)
   SS6: codierende Region der unter SEQ ID No. 1 angegebenen Nucleotidsequenz
   NOS: nos-Terminator (Depicker et al., J. Mol. Appl. Genet. 1, (1982), 561-573)
   RB: T-DNA right border
   Ampicillin: Ampicillin-Resistenzgen (Yanisch-Perron et al., Gene 33, (1985), 103-119)

In den Beispielen verwendete Medien und Lösungen:

| | |
|---|---|
| 20xSSC | 175,3 g NaCl. |
| | 88,2 g Natrium-Citrat |
| | ad 1000 ml mit ddH₂O |
| | pH 7,0 mit 10 N NaOH |
| | |
| YT | 8g Bacto-Yeast extract |
| | 5 g Bacto-Tryptone |
| | 5 g NaCI |
| | ad 1000 ml mit ddH₂O |

Protoplastenisolierungsmedium (100 ml)

| | |
|---|---|
| Cellulase Onozuka R S (Meiji Seika, Japan) | 800 mg |
| Pectolyase Y 23 | 40 mg |
| KNO₃ | 200 mg |
| KH₂PO₄ | 136 mg |
| K₂HPO₄ | 47 mg |
| CaCl₂2H₂O | 147 mg |
| MgSO₄ 7H₂O | 250 mg |
| Bovine serum albumin (BSA) | 20 mg : |
| Glucose | 4000 mg |
| Fructose | 4000 mg |
| Saccharose | 1000 mg |
| pH | 5,8 |
| Osmolarität | 660 mosm. |

Protoplastenwaschlösung 1: wie Protoplastenisolierlösung, aber ohne Cellulase, Pectolyase und BSA

### Transformationspuffer

a)

| | |
|---|---|
| Glucose | 0,5 M |
| MES | 0,1 % |
| MgCl₂ 6H₂0 | 25 mM |
| pH | 5,8 |
| auf 600 mosm. einstellen | |

b)

| | |
|---|---|
| PEG 6000-Lösung | |
| Glucose | 0,5 M |
| MgCl₂ 6H₂O | 100 mM |
| Hepes | 20 mM |
| pH | 6,5 |

Dem obigen Puffer unter b) wird PEG 6000 kurz vor Gebrauch der Lösung zugesetzt (40 Gew.-% PEG). Die Lösung wird durch ein 0,45 µm Sterilfilter filtriert.

| | |
|---|---|
| W5 Lösung | |
| CaCl₂ | 125 mM |
| NaCl | 150 mM |
| KCl | 5 mM |
| Glucose | 50 mM |

Protoplasten-Kulturmedium (Angaben in mg/l)

| | |
|---|---|
| KNO₃ | 3000 |
| (NH₄)₂SO₄ | 500 |
| MgSO₄ 7H₂O | 350 |
| KH₂PO₄ | 400 |
| CaCl₂ 2H₂O | 300 |

Fe-EDTA und Spurenelemente wie im Murashige-Skoog-Medium (Physiol. Plant, 15 (1962), 473).

| | |
|---|---|
| m-Inosit | 100 |
| Thiamin HCl | 1,0 |
| Nicotinsäureamid | 0,5 |
| Pyridoxin HCI | 0,5 |
| Glycin | 2,0 |
| Glucuronsäure | 750 |
| Galacturonsäure | 750 |
| Galactose | 500 |
| Maltose | 500 |
| Glucose | 36.000 |
| Fructose | 36.000 |
| Saccharose | 30.000 |
| Asparagin | 500 |
| Glutamin | 100 |
| Prolin | 300 |
| Caseinhydrolysat | 500 |
| 2,4-Dichlorphenoxyessigsäure (2,4-D) | 0,5 |
| pH | 5,8 |
| Osmolarität | 600 mosm. |

In den Beispielen werden die folgenden Methoden verwendet:

### 1. Transformation von Mais

(a) Herstellung von Protoplasten der Zellinie DSM 6009
   Protoplastenisolierung
   2 - 4 Tage, vorzugsweise 3 Tage nach dem letzten Mediumswechsel einer Protoplastensuspensionskultur wird das Flüssigmedium abgesaugt und die zurückbleibenden Zellen mit 50 ml Protoplastenwaschlösung 1 gespült und nochmals trockengesaugt. Zu jeweils 2 g der geernteten Zellmasse wird 10 ml Protoplastenisolierungsmedium gegeben. Die resuspendierten Zellen und Zellaggregate werden bei 27 ± 2° C unter leichtem Schütteln (30 bis 40 rpm) 4 bis 6 h im Dunkeln inkubiert.
   Protoplastenreinigung Sobald die Freisetzung von mindestens 1 Mio. Protoplasten/ml erfolgt ist (mikroskopische Beobachtung), wird die Suspension durch ein Edelstahl- und Nylonsieb von 200 bzw. 45 µm Maschenwerte gesiebt. Die Kombination eines 100 µm und eines 60 µm Siebs ermöglicht die Abtrennung der Zellaggregate genauso gut. Das protoplastenhaltige Filtrat wird mikroskopisch beurteilt. Üblicherweise enthält es 98 - 99 % Protoplasten. Der Rest sind unverdaute Einzelzellen. Protoplastenpräparationen mit diesem Reinheitsgrad werden ohne zusätzliche Gradientenzentrifugation für Transformationsexperimente verwendet. Durch Zentrifugation (100 UpM im Ausschwingrotor (100 x g, 3 min) werden die Protoplasten sedimentiert. Der Überstand wird verworfen und die Protoplasten in Waschlösung 1 resuspendiert: Die Zentrifugation wird wiederholt und die Protoplasten danach im Transformationspuffer resuspendiert.
(b) Protoplastentransformation
   Die in Tranformationspuffer resuspendierten Protoplasten werden bei einem Titer von 0,5 - 1 x 10⁶ Protoplasten/ml in 10 ml Portionen in 50 ml Polyallomer-Röhrchen eingefüllt. Die zur Transformation verwendete DNA wird in Tris-EDTA (TE) Puffer gelöst. Pro ml Protoplastensuspension werden 20 µg Plasmid-DNA zugegeben. Als Vektor wird dabei ein Phosphinothricinresistenz vermittelndes. Plasmid verwendet (vgl. z.B. EP-A2-0513 849). Nach der DNA-Zugabe wird die Protoplastensuspension vorsichtig geschüttelt, um die DNA homogen in der Lösung zu verteilen. Sofort danach wird tropfenweise 5 ml PEG-Lösung zugetropft. Durch vorsichtiges Schwenken der Röhrchen wird die PEG-Lösung homogen verteilt. Danach werden nochmals 5 ml PEG-Lösung zugegeben und das homogene Durchmischen wiederholt. Die Protoplasten verbleiben 20 min in der PEG-Lösung bei ± 2° C. Danach werden die Protoplasten durch 3-minütiges Zentrifugieren (100 g; 1000 Upm) sedimentiert. Der Überstand wird verworfen. Die Protoplasten werden durch vorsichtiges Schütteln in 20 ml W5-Lösung gewaschen und danach erneut zentrifugiert. Danach werden sie in 20 ml Protoplastenkulturmedium resuspendiert, nochmals zentrifugiert und erneut in Kulturmedium resuspendiert. Der Titer wird auf 6 - 8 x 10⁵ Protoplasten/ml eingestellt und die Protoplasten in 3 ml Portionen in Petrischalen (ø 60 mm, Höhe 15 mm) kultiviert. Die mit Parafilm versiegelten Petrischalen werden bei 25 ± 2° C im Dunkeln aufgestellt.
(c) Protoplastenkultur
   Während der ersten 2 - 3 Wochen nach der Protoplastenisolierung und - transformation werden die Protoplasten ohne Zugabe von frischem Medium kultiviert. Sobald sich die aus den Protoplasten regenerierten Zellen zu Zellaggregaten mit mehr als 20 - 50 Zellen entwickelt haben, wird 1 ml frisches Protoplastenkulturmedium zugegeben, das als Osmoticum Saccharose (90 g/l) enthält.
(d) Selektion transformierter Maiszellen und Pflanzenregeneration
   3 - 10 Tage nach der Zugabe von frischem Medium können die aus Protoplasten entstandenen Zellaggregate auf Agar-Medien mit 100 mg/l L-Phosphinothricin plattiert werden. N6-Medium mit den Vitaminen de's Protoplastenkulturmediums, 90 g/l Saccharose und 1,0 mg/l 2,4-D ist ebenso geeignet wie ein analoges Medium beispielsweise mit den Makro- und Mikronährsalzen des MS-Mediums (Murashige und Skoog (1962), siehe oben).
   Auf dem Selektivmedium können die aus stabil transformierten Protoplasten hervorgegangenen Kalli ungehindert weiterwachsen. Nach 3 - 5 Wochen, vorzugsweise 4 Wochen können die transgenen Kalli auf frisches Selektionsmedium transferiert werden, welches ebenfalls 100 mg/l L-Phosphinothricin enthält, das aber kein Auxin mehr enthält. Innerhalb von 3-5 Wochen differenzieren ca. 50 % der transgenen Maiskalli, die das L-Phosphinothricinacetyltransferase-Gen in ihr Genom integriert haben, auf diesem Medium in Gegenwart von L-Phosphinothricin erste Pflanzen.
(e) Aufzucht transgener Regeneratpflanzen
   Das embryogene transformierte Maisgewebe wird auf hormonfreiem N6-Medium (Chu C.C. et al., Sci. Sin. 16 (1975), 659) in Gegenwart von 5x10⁻⁴ M L-Phosphinothricin kultiviert. Auf diesem Medium entwickeln sich Maisembryonen, die das Phosphinothricinacetyltransferase-Gen (PAT-Gen) hinreichend stark exprimieren, zu Pflanzen. Nicht transformierte Embryonen oder solche mit nur sehr schwacher PAT-Aktivität sterben ab. Sobald die Blätter der in-Vitro-Pflanzen eine Länge von 4 - 6 mm erreicht haben, können diese in Erde transferiert werden. Nach Abwaschen von Agarresten an den Wurzeln werden die Pflanzen in ein Gemisch von Lehm, Sand, Vermiculit und Einheitserde im Verhältnis 3:1:1:1 gepflanzt und während der ersten 3 Tage nach dem Verpflanzen bei 90 - 100 % relativer Luftfeuchte an die Erdkultur adaptiert. Die Anzucht erfolgt in einer Klimakammer mit 14 h Lichtperiode ca. 25000 Lux in Pflanzenhöhe bei einer Tag/Nachttemperätur von 23 ± 1/17 ± 1° C. Die adaptierten Pflanzen werden bei einer Luftfeuchte von 65 ± 5 % kultiviert.

### 4. Radioaktive Markierung von DNA-Fragmenten

Die radioaktive Markierung von DNA-Fragmenten wurde mit Hilfe eines DNA-Random Primer Labelling Kits der Firma Boehringer (Deutschland) nach den Angaben des Herstellers durchgeführt.

Die folgenden Beispiele erläutern die Erfindung ohne sie in irgendeinerweise zu beschränken:

### Beispiel 1: Identifizierung, Isolierung und Charakterisierung einer cDNA, die eine neue Isoform einer Stärkesynthase aus Zea mays codiert

Zur Identifizierung einer cDNA codierend für eine neue Isoform der Stärkesynthase aus Zea mays wurde zunächst Gesamt-RNA aus Maiskörnern (15 bis 20 Tage nach der Bestäubung) nach der Methode von Logemann et al. (Anal. Biochem. 163, (1987), 21-26) isoliert. 1 mg Gesamt-RNA wurde anschließend verwendet, um unter Verwendung des Oligotex-mRNA- Reinigungs-Kits (Quiagen) nach Herstellerangaben poly A+-RNA zu präparieren.
Ausgehend von 5µg poly A+-RNA wurde anschließend mit Hilfe des ZAP-cDNA-Synthese- Kit der Firma Stratagene eine cDNA-Bibliothek konstruiert. Die Bibliothek enthielt ca. 9 x 10⁵ unabhängige rekombinante Phagen mit einer durchschnittlichen cDNA-Insert-Größe von ca. 1.3 kb.
Anschließend wurde das sogenannnte "Plaque-Lifting" an ca. 4x 10⁵ Phagen durchgeführt. Hierzu wurden Hybond N Filter (Amersham) verwendet. Nach einer 4-stündigen Prähybridisierung bei 42°C in Puffer A (5 x SSC, 0,5 % BSA, 5 x Denhardt, 1 % SDS, 40 mM Phosphatpuffer, pH 7.2; 100 mg/l Heringssperma-DNA, 25 % Formamid) wurden die Filter hybridisiert mit einem radioaktiv markierten (Random-Primed-DNA-Labeling-Kit, Boehringer Mannheim) EcoRl/Xhol-Fragment (gesamte cDNA) der SSIII-cDNA aus Solanum tuberosum (GenBank Acc. No. X 94400). Nach 12 h wurden die Filter dreimal für je 20 Minuten bei 55°C in einem Puffer enthaltend 3 x SSC, 0.5% SDS gewaschen. Anschließend wurde ein Röntgenfilm für ca. 14 h aufgelegt.
Stark hybridisierende Plaques wurden isoliert, verdünnt, nochmals ausplattiert und auf Filter, transferiert. Diese wurden erneut in der oben beschriebenen Weise hybridisiert und gewaschen. Nach in-vivo excision der isolierten Phagen nach Herstellerangaben (Stratagene) wurden verschiedene Plasmide isoliert, die mittels einer Restriktionsanalyse charakterisiert wurden. Anschließend wurden die DNA-Sequenzen der Plasmide bestimmt, die die längsten cDNA-Insertionen aufwiesen.
Eines dieser Plasmide, das als pZm_SS6 bezeichnet wurde enthielt die in SEQ ID NO. 1 angegebene Nucleotidsequenz.

### Beispiel 2 Herstellung eines Vektors zur Transformation von Pflanzen

Ausgehend von einem Notl/Bsp 120 1-Fragment des Plasmids pZm_SS6, das die vollständige codierende Region der unter SEQ ID No. 1 angegebenen Nucleotidsequenz enthält, wurde der Vektor IR65/87 (s. Figur 1), der u.a. geeignet ist zur Herstellung transgener Maispflanzen, die eine erhöhte Aktivität (Überexpressionsansatz) oder eine verringerte Aktivität (Cosuppressionsansatz) der erfindungsgemäßen Stärkesynthase aufweisen, mittels Standardverfahren hergestellt und bei der Deutschen Sammlung für Mikroorganismen am 5. August 1999 unter der Hinterfegungsnummer DSM 12970 hinterlegt.
Der Vektor IR65/87 wurde zur Transformation von Maispflanzen nach den oben beschriebenen Methoden verwendet.
Anschließend wurden transgene Pflanzen selektioniert, die entweder eine erhöhte Aktivität oder eine verringerte Aktivität des erfindungsgemäßen Proteins im Vergleich zu entsprechenden nicht-transformierten Maispflanzen aufwiesen.

## Patentansprüche

1. Nukleinsäuremolekül codierend ein Protein mit der biologischen Aktivität einer Stärkesynthase ausgewählt aus der Gruppe bestehend aus
(a) Nukleinsäuremolekülen, die ein Protein mit der unter Seq ID No. 2 angegebenen Aminosäuresequenz codieren;
(b) Nukleinsäuremolekülen, die die unter Seq ID No. 1 dargestellte Nucleotidsequenz umfassen;
(c) Nukleinsäuremolekülen, die die codierende Region der Nucleotidsequenz der im Plasmid IR 65/87, das unter der Hinterlegungsnummer DSM 12970 hinterlegt wurde, enthaltenen cDNA umfassen;
(d) Nukleinsäuremolekülen, deren Nucleotidsequenz aufgrund der Degeneration des genetisches Codes von der Sequenz der unter (a), (b) oder (c) genannten Nukleinsäuremoleküle abweicht;
(e) Nukleinsäuremolekülen, deren Sequenz zu über 85% identisch ist mit der codierenden Region der unter SEQ ID NO: 1 angegebenen Nukleotidsequenz; und
(f) Nukleinsäuremolekülen, deren Sequenz zu über 70% identisch ist mit der codierenden Region der unter SEQ ID NO: 1 angegebenen Nukleotidsequenz und die ein Protein codieren, dessen N-terminale Aminosäuren 1 bis 150 eine Sequenzidentität von mindestens 65% aufweisen zu der Aminosäuresequenz bestehend aus den Aminosäuren 1 bis 150 von SEQ ID NO: 2.

2. Nukleinsäuremolekül nach Anspruch 1, das ein DNA-Molekül ist.

3. Nukleinsäuremolekül nach Anspruch 1, das ein RNA-Molekül ist.

4. Vektor enthaltend ein Nukleinsäuremolekül nach einem oder mehreren der Ansprüche 1 bis 3.

5. Vektor nach Anspruch 4, enthaltend eines oder mehrere regulatorische Elemente, die die Transkription besagter Nukleinsäuremoleküle und/oder die Synthese einer translatierbaren RNA in einer pro- und/oder eukaryotischen Zelle gewährleisten.

6. Vektor nach einem oder mehreren der Ansprüche 4 bis 5, worin besagtes Nukleinsäuremolekül in sense-Orientierung mit regulatorischen Elementen verknüpft ist, die die Transkription und Synthese einer translatierbaren RNA in pro- und/oder eukaryontischen Zellen gewährleisten, oder worin besagtes Nukleinsäuremolekül in anti-sense-Orientierung mit regulatorischen Elementen verknüpft ist, die die Transkription und Synthese einer nicht translatierbaren RNA in pro- und/oder eukaryontischen Zellen gewährleisten.

7. Bakterielle oder pflanzliche Wirtszelle, die durch die Einführung eines Nukleinsäuremoleküls nach einem oder mehreren der Ansprüche 1 bis 3 oder eines Vektors nach einem oder mehreren der Ansprüche 4 bis 6 genetisch modifiziert ist.

8. Wirtszelle nach Anspruch 7, die eine pflanzliche Zelle ist.

9. Protein codiert durch ein Nukleinsäuremolekül nach einem oder mehreren der Ansprüche 1 bis 3.

10. Verfahren zur Herstellung eines Proteins nach Anspruch 9, bei dem eine Wirtszelle nach einem oder mehreren der Ansprüche 7 bis 8 unter Bedingungen kultiviert wird, die die Synthese des Proteins erlauben, und das Protein aus den kultivierten Zellen und/oder dem Kulturmedium isoliert wird.

11. Transgene Pflanzenzelle nach Anspruch 8, worin besagtes Nukleinsäuremolekül, das ein Protein mit der biologischen Aktivität einer Stärkesynthase codiert, unter der Kontrolle regulatorischer Elemente steht, die die Transkription einer translatierbaren mRNA in pflanzlichen Zellen erlauben.

12. Transgene Pflanzenzelle nach Anspruch 8, **dadurch gekennzeichnet, daß** in dieser Pflanzenzelle die Aktivität eines Proteins nach Anspruch 9 verringert ist im Vergleich zu entsprechenden nicht genetisch modifizierten Pflanzenzellen aus Wildtyppflanzen

13. Transgene Pflanzenzelle nach Anspruch 8, **dadurch gekennzeichnet, daß** in dieser Pflanzenzelle die Aktivität eines Proteins nach Anspruch 9 erhöht ist im Vergleich zu entsprechenden nicht genetisch modifizierten Pflanzenzellen aus Wildtyppflanzen.

14. Pflanze enthaltend Pflanzenzellen nach einem oder mehreren der Ansprüche 8 und 11 bis 13.

15. Pflanze nach Anspruch 14, die eine Nutzpflanze ist.

16. Pflanze nach einem oder mehreren der Ansprüche 14 bis 15, die eine stärkespeichernde Pflanze ist.

17. Pflanze nach einem oder mehreren der Ansprüche 14 bis 16, die eine Maispflanze ist.

18. Pflanze nach einem oder mehreren der Ansprüche 14 bis 17, **dadurch gekennzeichnet, dass** der Stärkeertrag um mindestens 10% erhöht ist im Vergleich zu nicht-transformierten Pflanzen desselben Genotyps.

19. Verfahren zur Herstellung einer transgenen Pflanzenzelle nach Anspruch 8, worin eine pflanzliche Zelle durch die Einführung eines Nukleinsäuremoleküls nach einem oder mehreren der Ansprüche 1 bis 3 und/oder eines Vektors nach einem oder mehreren der Ansprüche 4 bis 6 genetisch modifiziert wird.

20. Verfahren zur Herstellung einer transgenen Pflanze nach Anspruch 14, worin
(a) eine pflanzliche Zelle durch die Einführung eines Nukleinsäuremoleküls nach einem oder mehreren der Ansprüche 1 bis 3 und/oder eines Vektors nach einem oder mehreren der Ansprüche 4 bis 6 genetisch modifiziert wird; und
(b) aus besagter Zelle eine Pflanze regeneriert wird; und gegebenenfalls
(c) aus der Pflanze nach (b) weitere Pflanzen erzeugt werden.

21. Vermehrungsmaterial einer Pflanze nach einem oder mehreren der Ansprüche 14 bis 18, enthaltend Pflanzenzellen nach einem oder mehreren der Ansprüche 8 und 11 bis 13.

22. Verfahren zur Herstellung einer modifizierten Stärke umfassend den Schritt der Extraktion der Stärke aus einer Pflanzenzelle nach einem oder mehreren der Ansprüche 8 und 11 bis 13, aus einer Pflanze nach einem oder mehreren der Ansprüche 14 bis 18 und/oder aus Vermehrungsmaterial nach Anspruch 21.

## Claims

1. A nucleic acid molecule encoding a protein having the biological activity of a starch synthase selected from the group consisting of
(a) nucleic acid molecules encoding a protein with the amino acid sequence shown under SEQ ID NO:2;
(b) nucleic acid molecules comprising the nucleotide sequence shown under SEQ ID No:1;
(c) nucleic acid molecules comprising the coding region of the nucleotide sequence of the cDNA contained in plasmid IR 65/87 which was deposited under accession number DSM 12970;
(d) nucleic acid molecules, the nucleotide sequence of which deviates from the sequence of the nucleic acid molecules indicated under (a), (b) or (c) due to degeneration of the genetic code;
(e) nucleic acid molecules, the sequence of which is more than 85% identical to the coding region of the nucleotide sequence shown under SEQ ID NO:1; and
(f) nucleic acid molecules, the sequence of which is more than 70% identical to the coding region of the nucleotide sequence shown under SEQ ID NO:1, coding a protein the N-terminal amino acids 1 to 150 of which have sequence identity of at least 65% to the amino acid sequence consisting of the amino acids 1 to 150 of SEQ ID NO:2.

2. The nucleic acid molecule according to claim 1, which is a DNA molecule.

3. The nucleic acid molecule according to claim 1, which is an RNA molecule.

4. A vector containing a nucleic acid molecule according to one or more of claims 1 to 3.

5. The vector according to claim 4 containing one or more regulatory elements which ensure the transcription of said nucleic acid molecules and/or the synthesis of a translatable RNA in a prokaryotic and/or eukaryotic cell.

6. A vector according to one or more of claims 4 to 5, wherein said nucleic acid molecule is linked in sense orientation to regulatory elements which ensure the transcription and synthesis of a translatable RNA in prokaryotic and/or eukaryotic cells, or wherein said nucleic acid molecule is linked in anti-sense orientation to regulatory elements which ensure the transcription and synthesis of a non-translatable RNA in prokaryotic and/or eukaryotic cells.

7. A bacterial or plant host cell which is genetically modified by the introduction of a nucleic acid molecule according to one or more of claims 1 to 3 or of a vector according to one or more of claims 4 to 6.

8. The host cell according to claim 7, which is a plant cell.

9. A protein encoded by a nucleic acid molecule according to one or more of claims 1 to 3.

10. A method for the production of a protein according to claim 9, wherein a host cell according to one or more of claims 7 to 8 is cultivated under conditions allowing the synthesis of the protein and wherein the protein is isolated from the cultivated cells and/or the culture medium.

11. The transgenic plant cell according to claim 8, wherein said nucleic acid molecule encoding a protein having the biological activity of a starch synthase is under the control of regulatory elements allowing the transcription of a translatable mRNA in plant cells.

12. The transgenic plant cell according to claim 8 **characterized in that**, in this plant cell, the activity of a protein according to claim 9 is decreased in comparison to corresponding non-genetically modified plant cells from wild-type plants.

13. The transgenic plant cell according to claim 8 **characterized in that**, in this plant cell, the activity of a protein according to claim 9 is increased in comparison to corresponding non-genetically modified plant cells from wild-type plants.

14. A plant containing plant cells according to one or more of claims 8 and 11 to 13.

15. The plant of claim 14, which is a crop plant.

16. A plant according to one or more of claims 14 to 15, which is a starch-storing plant.

17. A plant according to one or more of claims 14 to 16, wherein said plant is a maize plant.

18. A plant according to one or more of claims 14 to 17 **characterized in that** the starch yield is increased by at least 10% in comparison to non-transformed plants of the same genotype.

19. A method for the production of a transgenic plant cell according to claim 8, wherein a plant cell is genetically modified by the introduction of a nucleic acid molecule according to one or more of claims 1 to 3 and/or of a vector according to one or more of claims 4 to 6.

20. The method for the production of a transgenic plant according to claim 14, wherein
(a) a plant cell is genetically modified by the introduction of a nucleic acid molecule according to one or more of claims 1 to 3 and/or of a vector according to one or more of claims 4 to 6; and
(b) a plant is regenerated from said cell; and optionally
(c) further plants are produced from the plant according to (b).

21. Propagation material of a plant according to one or more of claims 14 to 18 containing plant cells according to one or more of claims 8 and 11 to 13.

22. A method for the production of a modified starch comprising the step of extracting the starch from a plant cell according to one or more of claims 8 and 11 to 13, from a plant according to one or more of claims 14 to 18 and/or from propagation material according to claim 21.

## Revendications

1. Molécule d'acide nucléique codant une protéine ayant l'activité biologique d'une amidon synthase choisie dans le groupe constitué par
(a) les molécules d'acide nucléique qui codent une protéine avec la séquence d'acides aminés indiquée sous la Seq ID No. 2 ;
(b) les molécules- d'acide nucléique qui comprennent la séquence nucléotidique représentée sous la Seq ID No. 1 ;
(c) les molécules d'acide nucléique qui comprennent la région codante de la séquence nucléotidique de l'ADNc contenu dans le plasmide IR 65/87, qui a été déposé sous le numéro de dépôt DSM 12970 ;
(d) les molécules d'acide nucléique dont la séquence nucléotidique s'écarte, en raison de la dégénérescence du code génétique, de la séquence des molécules d'acide nucléique citées sous (a), (b) ou (c) ;
(e) les molécules d'acide nucléique dont la séquence est identique à plus de 85 % à la région codante de la séquence nucléotidique indiquée sous SEQ ID NO: 1 ; et
(f) les molécules d'acide nucléique dont la séquence est identique à plus de 70 % à la région codante de la séquence nucléotidique indiquée sous la SEQ ID NO: 1 et qui codent une protéine, dont les acides aminés 1 à 150 N-terminaux présentent une identité de séquence d'au moins 65 % à la séquence d'acides aminés constituée par les acides aminés 1 à 150 de la SEQ ID NO: 2.

2. Molécule d'acide nucléique selon la revendication 1, qui est une molécule d'ADN.

3. Molécule d'acide nucléique selon la revendication 1, qui est une molécule d'ARN.

4. Vecteur contenant une molécule d'acide nucléique selon une ou plusieurs des revendications 1 à 3.

5. Vecteur selon la revendication 4, contenant un ou plusieurs éléments régulateurs, qui assurent la transcription desdites molécules d'acide nucléique et/ou la synthèse d'un ARN traduisible dans une cellule pro- et/ou eucaryote.

6. Vecteur selon une ou plusieurs des revendications 4 à 5, dans lequel ladite molécule d'acide nucléique dans l'orientation sens est reliée à des éléments régulateurs, qui assurent la transcription et la synthèse d'un ARN traduisible dans des cellules pro- et/ou eucaryotes, ou dans lequel ladite molécule d'acide nucléique en orientation anti-sens est reliée à des éléments régulateurs qui assurent la transcription et la synthèse d'un ARN non traduisible dans des cellules pro- et/ou eucaryotes.

7. Cellule hôte bactérienne ou de plante qui est génétiquement modifiée par l'introduction d'une molécule d'acide nucléique selon une ou plusieurs des revendications 1 à 3 ou d'un vecteur selon une ou plusieurs des revendication 4 à 6.

8. Cellule hôte selon la revendication 7, qui est une cellule de plante.

9. Protéine codée par une molécule d'acide nucléique selon une ou plusieurs de revendications 1 à 3.

10. Procédé pour la préparation d'une protéine selon la revendication 9, dans lequel une cellule hôte selon une ou plusieurs des revendications 7 à 8 est cultivée dans des conditions qui permettent la synthèse de la protéine, et la protéine est isolée à partir des cellules cultivées et/ou du milieu de culture.

11. Cellule de plante transgénique selon la revendication 8, où ladite molécule d'acide nucléique, qui code une protéine ayant l'activité biologique d'une amidon synthase, est sous le contrôle d'éléments régulateurs qui permettent la transcription d'un ARNm traduisible dans les cellules de plante.

12. Cellule de plante transgénique selon la revendication 8, **caractérisée en ce que** dans cette cellule de plante l'activité d'une protéine selon la revendication 9 est réduite par comparaison aux cellules de plante correspondantes génétiquement non modifiées à partir de plantes de type sauvage.

13. Cellule de plante transgénique selon la revendication 8, **caractérisée en ce que** dans cette cellule de plante l'activité d'une protéine selon la revendication 9 est augmentée par comparaison aux cellules de plantes correspondantes génétiquement non modifiées à partir de plantes de type sauvage.

14. Plante contenant des cellules de plante selon une ou plusieurs des revendications 8 et 11 à 13.

15. Plante selon la revendication 14, qui est une plante utile.

16. Plante selon une ou plusieurs des revendications 14 à 15, qui est une plante de stockage de l'amidon.

17. Plante selon une ou plusieurs des revendications 14 à 16, qui est une plante de maïs.

18. Plante selon une ou plusieurs des revendications 14 à 17, **caractérisée en ce que** le rendement en amidon est augmenté d'au moins 10 % par comparaison aux plantes non transformées du même génotype.

19. Procédé pour la préparation d'une cellule de plante transgénique selon la revendication 8, dans lequel une cellule de plante est génétiquement modifiée par l'introduction d'une molécule d'acide nucléique selon une ou plusieurs des revendications 1 à 3 et/ou d'un vecteur selon une ou plusieurs des revendications 4 à 6.

20. Procédé pour la préparation d'une plante transgénique selon la revendication 14, dans lequel
(a) une cellule de plante est génétiquement modifiée par l'introduction d'une molécule d'acide nucléique selon une ou plusieurs des revendications 1 à 3 et/ou d'un vecteur selon une ou plusieurs des revendications 4 à 6 ; et
(b) une plante est régénérée à partir de ladite cellule ; et éventuellement
(c) d'autres plantes sont produites à partir de ladite plante selon (b).

21. Matière de multiplication d'une plante selon une ou plusieurs des revendications 14 à 18, contenant des cellules de plante selon une ou plusieurs des revendications 8 et 11 à 13.

22. Procédé pour la préparation d'un amidon modifié comprenant l'étape de l'extraction de l'amidon à partir d'une cellule de plante selon une ou plusieurs des revendications 8 et 11 à 13, à partir d'une plante selon une ou plusieurs des revendications 14 à 18 et/ou à partir de la matière de multiplication selon la revendication 21.


### SEQUENCE LISTING

<110> Aventis CropScience GmbH
<120> Nukleinsäuremoleküle aus Pflanzen codierend Enzyme, die an der Stärkesynthese beteiligt sind
<130> AGR 1999/M 225
<140> 19 937 348.5
   <141> 1999-08-11
<160> 29
<170> PatentIn Ver. 2.1
<210> 1
   <211> 4121
   <212> DNA
   <213> Zea mays
<220>
   <221> CDS
   <222> (442)..(3954)
<400> 1
   gaattcggca cgagccgctt tggatccacc tctctgcgcg cggggccgcg cctgcacttc 60
   gcacagtggc aggcggcagc ccccaacgcc atccacacat cccccggcaa aatccttacc 120
   caccgaaaaa agggaaaaat ggaggcaggc aggcggcact gcggcagtca cacatgcgca 180
   cgggcacggt aaaagtacga gccacctttt ccctctcgat cgagcggccg aggaggagga 240
   gagcctctct gtctctggcg aggggcgact gggcgagcgc ttcgtctaga cagcccagct 300
   gcgcatttgg atgcaaatta cgtgtggagg atggagtaaa atactgagca agggagaggg 360
   aagaggagac ggtcttttct ttcttgagcc cgaggagggg gatcaggtgg agtgagcgag 420
   ggggaggttt tgccgccggc a atg gag atg aat ctc cgg gcg gag agc cca 471
                           Met Glu Met Asn Leu Arg Ala Glu Ser Pro
                           1               5                   10
   ctc tgc tcc cgg ggc cgc ccc gcc ctc gtc gtg cgc ccg gcc gct gca 519
   Leu Cys Ser Arg Gly Arg Pro Ala Leu Val Val Arg Pro Ala Ala Ala
                   15                  20                  25
   gcc act ggc ctc gcg ctg tct gtc gta agg tgc agc aga ttt acg aga 567
   Ala Thr Gly Leu Ala Leu Ser Val Val Arg Cys Ser Arg Phe Thr Arg
               30                  35                  40
   ggc ggg ctc gtt cga tgc atg gta tca agt tca gat tat ccc aag agg 615
   Gly Gly Leu Val Arg Cys Met Val Ser Ser Ser Asp Tyr Pro Lys Arg
           45                  50                  55
   aat ccg agg agg gca tcg act tct aag agc aag ggc gtt gcc tct gga 663
   Asn Pro Arg Arg Ala Ser Thr Ser Lys Ser Lys Gly Val Ala Ser Gly
       60                  65                  70
   ggg tat gct tcg aga cct act gct gaa tcc agt acg aag aag ata gaa 711
   Gly Tyr Ala Ser Arg Pro Thr Ala Glu Ser Ser Thr Lys Lys Ile Glu
   75                  80                  85                  90
   cag agc agg aac aat gaa ggt gat ttc agc aga gcc aat ggg tca ctc    759
   Gln Ser Arg Asn Asn Glu Gly Asp Phe Ser Arg Ala Asn Gly Ser Leu
                    95                 100                 105
   tat ggc gag gca gca gag cag gca agt act gct gaa gaa tcg tct cag    807
   Tyr Gly Glu Ala Ala Glu Gln Ala Ser Thr Ala Glu Glu Ser Ser Gln
               110                 115                 120
   gtt tac atg aca gga gac att tta agt ggc gca gaa agg gac gga gct    855
   Val Tyr Met Thr Gly Asp Ile Leu Ser Gly Ala Glu Arg Asp Gly Ala
           125                 130                 135
   ggt acc gaa gaa gag gct gac caa aat caa tct tca gcg ttg cct tcc    903
   Gly Thr Glu Glu Glu Ala Asp Gln Asn Gln Ser Ser Ala Leu Pro Ser
       140                 145                 150
   gcg tcc atg gat gat gac tca atc gac cgg caa ctt gat gag tac cgt    951
   Ala Ser Met Asp Asp Asp Ser Ile Asp Arg Gln Leu Asp Glu Tyr Arg
   155                 160                 165                 170
   ggt aaa ata agt gct cta gta agt tcc aaa cct gaa cct tcg tca ctt    999
   Gly Lys Ile Ser Ala Leu Val Ser Ser Lys Pro Glu Pro Ser Ser Leu
                   175                 180                 185
   gca agt gtt gct gga caa aac gaa tca gtt ggc ggt ttt cac ggc cag    1047
   Ala Ser Val Ala Gly Gln Asn Glu Ser Val Gly Gly Phe His Gly Gln
               190                 195                 200
   cac gaa cca ata act ggt tct gag gaa cat ggc agt tca att gtt gat    1095
   His Glu Pro Ile Thr Gly Ser Glu Glu His Gly Ser Ser Ile Val Asp
           205                 210                 215
   gca cca ata aaa ggc agg cta ttt gct gag gcc gtt gtg ggt cat aag    1143
   Ala Pro Ile Lys Gly Arg Leu Phe Ala Glu Ala Val Val Gly His Lys
       220                 225                 230
   gat ttt act gaa tca gca gca gga aag gca agc agc gag aat gag gaa    1191
   Asp Phe Thr Glu Ser Ala Ala Gly Lys Ala Ser Ser Glu Asn Glu Glu
   235                 240                 245                 250
   ggg caa gct gtc tcg tta gaa gat gat gtc ggg ata agt aca gac gca    1239
   Gly Gln Ala Val Ser Leu Glu Asp Asp Val Gly Ile Ser Thr Asp Ala
                   255                 260                 265
   gat gaa gag ctt ccg gta tct gaa gat gat cca gaa gtg cta ctg agg    1287
   Asp Glu Glu Leu Pro Val Ser Glu Asp Asp Pro Glu Val Leu Leu Arg
               270                 275                 280
   agg ctt caa gag ctt gct gat gag aat tat tcg act ggg aac aac tgt    1335
   Arg Leu Gln Glu Leu Ala Asp Glu Asn Tyr Ser Thr Gly Asn Asn Cys
           285                 290                 295
   ttt gtt ttc cct gaa gta gtg aag gct gat tcg atg att gat ctt tac    1383
   Phe Val Phe Pro Glu Val Val Lys Ala Asp Ser Met Ile Asp Leu Tyr
       300                 305                 310
   tta aac cgc agc atg tcg gcc tta gct agt gag tcc gac gtt ttt gta    1431
   Leu Asn Arg Ser Met Ser Ala Leu Ala Ser Glu Ser Asp Val Phe Val
   315                 320                 325                 330
   aaa gga gca ttc aat ggt tgg aga tgg aac cgt ttc act gaa aca atg    1479
   Lys Gly Ala Phe Asn Gly Trp Arg Trp Asn Arg Phe Thr Glu Thr Met
                   335                 340                 345
   cat aga agc gaa tta aga ggg gat tgg tgg tgc tgc aag ctc tac att   1527
   His Arg Ser Glu Leu Arg Gly Asp Trp Trp Cys Cys Lys Leu Tyr Ile
               350                 355                 360
   ccc aag cag gca tac aga cta gac ttt gta ttc ttt aac ggt gac act   1575
   Pro Lys Gln Ala Tyr Arg Leu Asp Phe Val Phe Phe Asn Gly Asp Thr
           365                 370                 375
   gtc tat gaa aat aac aat cac aac gat ttt ttc ctg gaa ata gaa agt   1623
   Val Tyr Glu Asn Asn Asn His Asn Asp Phe Phe Leu Glu Ile Glu Ser
       380                 385                 390
   gac ata gat gaa cac tca ttt gag gac ttc ttg gtt gaa gaa aag cga   1671
   Asp Ile Asp Glu His Ser Phe Glu Asp Phe Leu Val Glu Glu Lys Arg
   395                 400                 405                 410
   aag gaa ctc gag agg ctt gct gca gaa gaa gct gaa agg aaa aga caa   1719
   Lys Glu Leu Glu Arg Leu Ala Ala Glu Glu Ala Glu Arg Lys Arg Gln
                   415                 420                 425
   gct gag gag cgg cac agg aga gag gaa gaa agg gcc gcg atg gaa gct   1767
   Ala Glu Glu Arg His Arg Arg Glu Glu Glu Arg Ala Ala Met Glu Ala
               430                 435                 440
   gac agg gca caa gca agg tct gag gtc gag atg aag aaa gag aaa ttg   1815
   Asp Arg Ala Gln Ala Arg Ser Glu Val Glu Met Lys Lys Glu Lys Leu
           445                 450                 455
   cgc cag atg ttg agt tcg gct agc aga tat gct gag aac tta tgg tac   1863
   Arg Gln Met Leu Ser Ser Ala Ser Arg Tyr Ala Glu Asn Leu Trp Tyr
       460                 465                 470
   ata gaa cct aac acc tac aga gga gga gac aga gtt aga ttg tac tat   1911
   Ile Glu Pro Asn Thr Tyr Arg Gly Gly Asp Arg Val Arg Leu Tyr Tyr
   475                 480                 485                 490
   aat aga agc tcg aga tca cta atg cat aac act gag att tgg atg cat   1959
   Asn Arg Ser Ser Arg Ser Leu Met His Asn Thr Glu Ile Trp Met His
                   495                 500                 505
   gga ggt tat aac aac tgg att gat gga ctc tca ata gct gaa aga ctt   2007
   Gly Gly Tyr Asn Asn Trp Ile Asp Gly Leu Ser Ile Ala Glu Arg Leu
               510                 515                 520
   gtc aaa tct cat gaa aag gaa ggc gat tgg tgg tat gta gaa gtt aca   2055
   Val Lys Ser His Glu Lys Glu Gly Asp Trp Trp Tyr Val Glu Val Thr
           525                 530                 535
   tta cct gaa agg gca ttg gtg ttg gat tgg gtt ttt gct gat gga cca   2103
   Leu Pro Glu Arg Ala Leu Val Leu Asp Trp Val Phe Ala Asp Gly Pro
       540                 545                 550
   cct ggg aat gca agg aat tat gat aac aat gga agg cag gat ttt cat   2151
   Pro Gly Asn Ala Arg Asn Tyr Asp Asn Asn Gly Arg Gln Asp Phe His
   555                 560                 565                 570
   gcc atc gtt cct aat aac ata tcg gat gat atc ttt tgg gtg gaa gaa   2199
   Ala Ile Val Pro Asn Asn Ile Ser Asp Asp Ile Phe Trp Val Glu Glu
                   575                 580                 585
   gaa cat agg atc ttt aca agg ctt caa caa gag aga aga gaa agg gag      2247
   Glu His Arg Ile Phe Thr Arg Leu Gln Gln Glu Arg Arg Glu Arg Glu
               590                 595                 600
   agt gcc gaa aga ata aag gct gag aga tct gca aaa atg aag gct gag      2295
   Ser Ala Glu Arg Ile Lys Ala Glu Arg Ser Ala Lys Met Lys Ala Glu
           605                 610                 615
   atg aag gaa aag act atg aga gcg ttt ctg ctc tca caa aaa cat att      2343
   Met Lys Glu Lys Thr Met Arg Ala Phe Leu Leu Ser Gln Lys His Ile
       620                 625                 630
   gtg tat act gag cca ctc gaa gta cgt gca gga acc act gtg gac gtt      2391
   Val Tyr Thr Glu Pro Leu Glu Val Arg Ala Gly Thr Thr Val Asp Val
   635                 640                 645                 650
   ctt tat aat cct tct aac aca gtg ctg aat gga aag tca gag gtt tgg      2439
   Leu Tyr Asn Pro Ser Asn Thr Val Leu Asn Gly Lys Ser Glu Val Trp
                   655                 660                 665
   ttc aga ggt tcc ttt aac cgt tgg act cat cca agt ggt ccc tta cca      2487
   Phe Arg Gly Ser Phe Asn Arg Trp Thr His Pro Ser Gly Pro Leu Pro
               670                 675                 680
   cca cag aag atg gta aag gct gag aat agt tca cac tta cga aca aca      2535
   Pro Gln Lys Met Val Lys Ala Glu Asn Ser Ser His Leu Arg Thr Thr
           685                 690                 695
   gtc agt gtt ccc ctg gat gca tat atg atg gac ttt gtt ttc tct gag      2583
   Val Ser Val Pro Leu Asp Ala Tyr Met Met Asp Phe Val Phe Ser Glu
       700                 705                 710
   tcg gaa gaa ggt gga aga tat gac aat agg aac ggg atg gat tat cat      2631
   Ser Glu Glu Gly Gly Arg Tyr Asp Asn Arg Asn Gly Met Asp Tyr His
   715                 720                 725                 730
   att cct gtg tct gat tcg gtt gca agg gaa cct cca atg cat att gta      2679
   Ile Pro Val Ser Asp Ser Val Ala Arg Glu Pro Pro Met His Ile Val
                   735                 740                 745
   cac att gca gtg gaa atg gct cct atc gca aag gtt gga ggc ctt ggt      2727
   His Ile Ala Val Glu Met Ala Pro Ile Ala Lys Val Gly Gly Leu Gly
               750                 755                 760
   gat gtt gtt aca agc ctt tca cga gct gtt cag gat tta ggc cat aaa      2775
   Asp Val Val Thr Ser Leu Ser Arg Ala Val Gln Asp Leu Gly His Lys
           765                 770                 775
   gtt gag gtt att ctg ccg aag tat gat tgt tta atc cta agc agt gtg      2823
   Val Glu Val Ile Leu Pro Lys Tyr Asp Cys Leu Ile Leu Ser Ser Val
       780                 785                 790
   aag gat tta cac tac caa caa agt ttt gct tcg ggt ggc aca gag gta      2871
   Lys Asp Leu His Tyr Gln Gln Ser Phe Ala Ser Gly Gly Thr Glu Val
   795                 800                 805                 810
   aaa gta tgg ttt gga aag gtt gaa gat ctg cca gtt tac ttc ttg gaa      2919
   Lys Val Trp Phe Gly Lys Val Glu Asp Leu Pro Val Tyr Phe Leu Glu
                   815                 820                 825
   cca caa aat ggc atg ttt tgg gtt gga tgt gtg tat ggg aag aat gat      2967
   Pro Gln Asn Gly Met Phe Trp Val Gly Cys Val Tyr Gly Lys Asn Asp
               830                 835                 840
   gag agt aga ttt ggc ttc ttc tgt cat tct gct ctg gag ttt ctg ctc      3015
   Glu Ser Arg Phe Gly Phe Phe Cys His Ser Ala Leu Glu Phe Leu Leu
           845                 850                 855
   caa aaa gga tct tct cct gat atc ata cat tgt cat gac tgg tca agt      3063
   Gln Lys Gly Ser Ser Pro Asp Ile Ile His Cys His Asp Trp Ser Ser
       860                 865                 870
   gct ccc gtt gct tgg cta tac aag gaa cag tat gct ctt aat ggg ctg      3111
   Ala Pro Val Ala Trp Leu Tyr Lys Glu Gln Tyr Ala Leu Asn Gly Leu
   875                 880                 885                 890
   gga aat ggt cgg att gta ttt acc atc cac aat ctt gag ttt gga gcg      3159
   Gly Asn Gly Arg Ile Val Phe Thr Ile His Asn Leu Glu Phe Gly Ala
                   895                 900                 905
   cat cac att ggc aag gca atg gca cat tgt gac aag gct aca act gtc      3207
   His His Ile Gly Lys Ala Met Ala His Cys Asp Lys Ala Thr Thr Val
               910                 915                 920
   tct gat aca tat tca aag gaa gtg gct gga cat gga gct att gca cct      3255
   Ser Asp Thr Tyr Ser Lys Glu Val Ala Gly His Gly Ala Ile Ala Pro
           925                 930                 935
   cac tac tat aaa ttc cat gga att cga aat gga att gat cct gac att      3303
   His Tyr Tyr Lys Phe His Gly Ile Arg Asn Gly Ile Asp Pro Asp Ile
       940                 945                 950
   tgg gat cca tat act gac aga ttt att ccg gtt cat tat aca tca gag      3351
   Trp Asp Pro Tyr Thr Asp Arg Phe Ile Pro Val His Tyr Thr Ser Glu
   955                 960                 965                 970
   aat gtt ctt gag ggc aag ggt gct gca aaa aag gca ttg cag cag atg      3399
   Asn Val Leu Glu Gly Lys Gly Ala Ala Lys Lys Ala Leu Gln Gln Met
                   975                 980                 985
   ctt gga tta cag caa act gat agc cct gtt gtt gga atc atc act cgt      3447
   Leu Gly Leu Gln Gln Thr Asp Ser Pro Val Val Gly Ile Ile Thr Arg
               990                 995                 1000
   cta aca gtg cag aag gga atc cac ctt atc aaa cat gca atg cat cga      3495
   Leu Thr Val Gln Lys Gly Ile His Leu Ile Lys His Ala Met His Arg
           1005                1010                1015
   gct ctt gaa cgc aat ggg cag gtg gtt tta ctg ggt tct gca cca gat      3543
   Ala Leu Glu Arg Asn Gly Gln Val Val Leu Leu Gly Ser Ala Pro Asp
       1020                1025                1030
   cat cgc ata caa ggt gac ttt aca aat tta gcc agt aag ctg cat ggt      3591
   His Arg Ile Gln Gly Asp Phe Thr Asn Leu Ala Ser Lys Leu His Gly
   1035                1040                1045                1050
   gaa tac cat ggc cgg gtg aag cta tgt tta acc tat gac gag cca ctg      3639
   Glu Tyr His Gly Arg Val Lys Leu Cys Leu Thr Tyr Asp Glu Pro Leu
                   1055                1060                1065
   tca cat ttg ctt att gct atg cgc tac gga tcc atc ccg att gtt cgg      3687
   Ser His Leu Leu Ile Ala Met Arg Tyr Gly Ser Ile Pro Ile Val Arg
               1070                1075                1080
   aaa act gga ggc ctg tac gac acc gtc ttt gac gtc gac gat gat aag   3735
   Lys Thr Gly Gly Leu Tyr Asp Thr Val Phe Asp Val Asp Asp Asp Lys
           1085                1090                1095
   gat cgg gct caa gca caa ggc ctc gag cca aat gga ttc agt ttc gaa   3783
   Asp Arg Ala Gln Ala Gln Gly Leu Glu Pro Asn Gly Phe Ser Phe Glu
       1100                1105                1110
   gga gct gat agc agt ggt gta gat tat gct ctc gac aga gct ata acc   3831
   Gly Ala Asp Ser Ser Gly Val Asp Tyr Ala Leu Asp Arg Ala Ile Thr
   1115                1120                1125                1130
   gcg ttt tat gat gcc cgc gac tgg ttc aac tcc ctt agc aag agg gta   3879
   Ala Phe Tyr Asp Ala Arg Asp Trp Phe Asn Ser Leu Ser Lys Arg Val
                   1135                1140                1145
   atg gag caa gat tgg tca tgg aat aga cct gca cta gac tac atg gag   3927
   Met Glu Gln Asp Trp Ser Trp Asn Arg Pro Ala Leu Asp Tyr Met Glu
               1150                1155                1160
   ttg tac cat tct gct cgc aaa aac tga tacattttac cacaagggaa         3974
   Leu Tyr His Ser Ala Arg Lys Asn
           1165                1170
   aacaggccca gtttgcccag tttatgagca tctcagatgc aacacagagt gtatagttag 4034
   agaatgccac ccgttgtacg ttactgccgt tacatgcatg tgtatacaca tctaaaaaaa 4094
   aaaaaaaaaa aacggcacga gctcgtg                                     4121
<210> 2
   <211> 1170
   <212> PRT
   <213> Zea mays
<400> 2
   Met Glu Met Asn Leu Arg Ala Glu Ser Pro Leu Cys Ser Arg Gly Arg
    1               5                  10                  15
   Pro Ala Leu Val Val Arg Pro Ala Ala Ala Ala Thr Gly Leu Ala Leu
               20                  25                  30
   Ser Val Val Arg Cys Ser Arg Phe Thr Arg Gly Gly Leu Val Arg Cys
           35                  40                  45
   Met Val Ser Ser Ser Asp Tyr Pro Lys Arg Asn Pro Arg Arg Ala Ser
       50                  55                  60
   Thr Ser Lys Ser Lys Gly Val Ala Ser Gly Gly Tyr Ala Ser Arg Pro
   65                  70                  75                  80
   Thr Ala Glu Ser Ser Thr Lys Lys Ile Glu Gln Ser Arg Asn Asn Glu
                   85                  90                  95
   Gly Asp Phe Ser Arg Ala Asn Gly Ser Leu Tyr Gly Glu Ala Ala Glu
               100                 105                 110
   Gln Ala Ser Thr Ala Glu Glu Ser Ser Gln Val Tyr Met Thr Gly Asp
           115                 120                 125
   Ile Leu Ser Gly Ala Glu Arg Asp Gly Ala Gly Thr Glu Glu Glu Ala
       130                 135                 140
   Asp Gln Asn Gln Ser Ser Ala Leu Pro Ser Ala Ser Met Asp Asp Asp
   145                 150                 155                 160
   Ser Ile Asp Arg Gln Leu Asp Glu Tyr Arg Gly Lys Ile Ser Ala Leu
                   165                 170                 175
   Val Ser Ser Lys Pro Glu Pro Ser Ser Leu Ala Ser Val Ala Gly Gln
               180                 185                 190
   Asn Glu Ser Val Gly Gly Phe His Gly Gln His Glu Pro Ile Thr Gly
           195                 200                 205
   Ser Glu Glu His Gly Ser Ser Ile Val Asp Ala Pro Ile Lys Gly Arg
       210                 215                 220
   Leu Phe Ala Glu Ala Val Val Gly His Lys Asp Phe Thr Glu Ser Ala
   225                 230                 235                 240
   Ala Gly Lys Ala Ser Ser Glu Asn Glu Glu Gly Gln Ala Val Ser Leu
                   245                 250                 255
   Glu Asp Asp Val Gly Ile Ser Thr Asp Ala Asp Glu Glu Leu Pro Val
               260                 265                 270
   Ser Glu Asp Asp Pro Glu Val Leu Leu Arg Arg Leu Gln Glu Leu Ala
           275                 280                 285
   Asp Glu Asn Tyr Ser Thr Gly Asn Asn Cys Phe Val Phe Pro Glu Val
       290                 295                 300
   Val Lys Ala Asp Ser Met Ile Asp Leu Tyr Leu Asn Arg Ser Met Ser
   305                 310                 315                 320
   Ala Leu Ala Ser Glu Ser Asp Val Phe Val Lys Gly Ala Phe Asn Gly
                   325                 330                 335
   Trp Arg Trp Asn Arg Phe Thr Glu Thr Met His Arg Ser Glu Leu Arg
               340                 345                 350
   Gly Asp Trp Trp Cys Cys Lys Leu Tyr Ile Pro Lys Gln Ala Tyr Arg
           355                 360                 365
   Leu Asp Phe Val Phe Phe Asn Gly Asp Thr Val Tyr Glu Asn Asn Asn
       370                 375                 380
   His Asn Asp Phe Phe Leu Glu Ile Glu Ser Asp Ile Asp Glu His Ser
   385                 390                 395                 400
   Phe Glu Asp Phe Leu Val Glu Glu Lys Arg Lys Glu Leu Glu Arg Leu
                   405                 410                 415
   Ala Ala Glu Glu Ala Glu Arg Lys Arg Gln Ala Glu Glu Arg His Arg
               420                 425                 430
   Arg Glu Glu Glu Arg Ala Ala Met Glu Ala Asp Arg Ala Gln Ala Arg
           435                 440                 445
   Ser Glu Val Glu Met Lys Lys Glu Lys Leu Arg Gln Met Leu Ser Ser
       450                 455                 460
   Ala Ser Arg Tyr Ala Glu Asn Leu Trp Tyr Ile Glu Pro Asn Thr Tyr
   465                 470                 475                 480
   Arg Gly Gly Asp Arg Val Arg Leu Tyr Tyr Asn Arg Ser Ser Arg Ser
                   485                 490                 495
   Leu Met His Asn Thr Glu Ile Trp Met His Gly Gly Tyr Asn Asn Trp
               500                 505                 510
   Ile Asp Gly Leu Ser Ile Ala Glu Arg Leu Val Lys Ser His Glu Lys
           515                 520                 525
   Glu Gly Asp Trp Trp Tyr Val Glu Val Thr Leu Pro Glu Arg Ala Leu
       530                 535                 540
   Val Leu Asp Trp Val Phe Ala Asp Gly Pro Pro Gly Asn Ala Arg Asn
   545                 550                 555                 560
   Tyr Asp Asn Asn Gly Arg Gln Asp Phe His Ala Ile Val Pro Asn Asn
                   565                 570                 575
   Ile Ser Asp Asp Ile Phe Trp Val Glu Glu Glu His Arg Ile Phe Thr
               580                 585                 590
   Arg Leu Gln Gln Glu Arg Arg Glu Arg Glu Ser Ala Glu Arg Ile Lys
           595                 600                 605
   Ala Glu Arg Ser Ala Lys Met Lys Ala Glu Met Lys Glu Lys Thr Met
       610                 615                 620
   Arg Ala Phe Leu Leu Ser Gln Lys His Ile Val Tyr Thr Glu Pro Leu
   625                 630                 635                 640
   Glu Val Arg Ala Gly Thr Thr Val Asp Val Leu Tyr Asn Pro Ser Asn
                   645                 650                 655
   Thr Val Leu Asn Gly Lys Ser Glu Val Trp Phe Arg Gly Ser Phe Asn
               660                 665                 670
   Arg Trp Thr His Pro Ser Gly Pro Leu Pro Pro Gln Lys Met Val Lys
           675                 680                 685
   Ala Glu Asn Ser Ser His Leu Arg Thr Thr Val Ser Val Pro Leu Asp
       690                 695                 700
   Ala Tyr Met Met Asp Phe Val Phe Ser Glu Ser Glu Glu Gly Gly Arg
   705                 710                 715                 720
   Tyr Asp Asn Arg Asn Gly Met Asp Tyr His Ile Pro Val Ser Asp Ser
                   725                 730                 735
   Val Ala Arg Glu Pro Pro Met His Ile Val His Ile Ala Val Glu Met
               740                 745                 750
   Ala Pro Ile Ala Lys Val Gly Gly Leu Gly Asp Val Val Thr Ser Leu
           755                 760                 765
   Ser Arg Ala Val Gln Asp Leu Gly His Lys Val Glu Val Ile Leu Pro
       770                 775                 780
   Lys Tyr Asp Cys Leu Ile Leu Ser Ser Val Lys Asp Leu His Tyr Gln
   785                 790                 795                 800
   Gln Ser Phe Ala Ser Gly Gly Thr Glu Val Lys Val Trp Phe Gly Lys
                   805                 810                 815
   Val Glu Asp Leu Pro Val Tyr Phe Leu Glu Pro Gln Asn Gly Met Phe
               820                 825                 830
   Trp Val Gly Cys Val Tyr Gly Lys Asn Asp Glu Ser Arg Phe Gly Phe
           835                 840                 845
   Phe Cys His Ser Ala Leu Glu Phe Leu Leu Gln Lys Gly Ser Ser Pro
       850                 855                 860
   Asp Ile Ile His Cys His Asp Trp Ser Ser Ala Pro Val Ala Trp Leu
   865                 870                 875                 880
   Tyr Lys Glu Gln Tyr Ala Leu Asn Gly Leu Gly Asn Gly Arg Ile Val
                   885                 890                 895
   Phe Thr Ile His Asn Leu Glu Phe Gly Ala His His Ile Gly Lys Ala
               900                 905                 910
   Met Ala His Cys Asp Lys Ala Thr Thr Val Ser Asp Thr Tyr Ser Lys
           915                 920                 925
   Glu Val Ala Gly His Gly Ala Ile Ala Pro His Tyr Tyr Lys Phe His
       930                 935                 940
   Gly Ile Arg Asn Gly Ile Asp Pro Asp Ile Trp Asp Pro Tyr Thr Asp
   945                 950                 955                 960
   Arg Phe Ile Pro Val His Tyr Thr Ser Glu Asn Val Leu Glu Gly Lys
                   965                 970                 975
   Gly Ala Ala Lys Lys Ala Leu Gln Gln Met Leu Gly Leu Gln Gln Thr
               980                 985                 990
   Asp Ser Pro Val Val Gly Ile Ile Thr Arg Leu Thr Val Gln Lys Gly
           995                 1000                1005
   Ile His Leu Ile Lys His Ala Met His Arg Ala Leu Glu Arg Asn Gly
       1010                1015                1020
   Gln Val Val Leu Leu Gly Ser Ala Pro Asp His Arg Ile Gln Gly Asp
   1025                1030                1035                1040
   Phe Thr Asn Leu Ala Ser Lys Leu His Gly Glu Tyr His Gly Arg Val
                   1045                1050                1055
   Lys Leu Cys Leu Thr Tyr Asp Glu Pro Leu Ser His Leu Leu Ile Ala
               1060                1065                1070
   Met Arg Tyr Gly Ser Ile Pro Ile Val Arg Lys Thr Gly Gly Leu Tyr
           1075                1080                1085
   Asp Thr Val Phe Asp Val Asp Asp Asp Lys Asp Arg Ala Gln Ala Gln
       1090                1095                1100
   Gly Leu Glu Pro Asn Gly Phe Ser Phe Glu Gly Ala Asp Ser Ser Gly
   1105                1110                1115                1120
   Val Asp Tyr Ala Leu Asp Arg Ala Ile Thr Ala Phe Tyr Asp Ala Arg
                   1125                1130                1135
   Asp Trp Phe Asn Ser Leu Ser Lys Arg Val Met Glu Gln Asp Trp Ser
               1140                1145                1150
   Trp Asn Arg Pro Ala Leu Asp Tyr Met Glu Leu Tyr His Ser Ala Arg
           1155                1160                1165
   Lys Asn
       1170
<210> 3
   <211> 32
   <212 > PRT
   <213> Artificial Sequence
<220>
   <223> Description of Artificial Sequence: motif VII
<400> 3
   Ser His Thr Ile Tyr Ala Ala Ser Asp Leu Phe Ile Ile Pro Ser Ile
   1               5                   10                  15
   Phe Glu Pro Cys Gly Leu Thr Gln Met Ile Ala Met Arg Tyr Gly Ser
               20                  25                  30
<210> 4
   <211> 32
   <212> PRT
   <213> Artificial Sequence
<220>
   <223> Description of Artificial Sequence: motif VII
<400> 4
   Ser His Leu Ile Tyr Ala Gly Ala Asp Phe Ile Leu Val Pro Ser Ile
   1               5                   10                  15
   Phe Glu Pro Cys Gly Leu Thr Gln Leu Thr Ala Met Arg Tyr Gly Ser
               20                  25                  30
<210> 5
   <211> 32
   <212> PRT
   <213> Artificial Sequence
<220>
   <223> Description of Artificial Sequence: motif VII
<400> 5
   Ser His Leu Ile Tyr Ala Gly Ala Asp Phe Ile Leu Val Pro Ser Ile
   1               5                   10                  15
   Phe Glu Pro Cys Gly Leu Thr Gln Leu Thr Ala Met Arg Tyr Gly Ser
               20                  25                  30
<210> 6
   <211> 32
   <212> PRT
   <213> Artificial Sequence
<220>
   <223> Description of Artificial Sequence: motif VII
<400> 6
   Ala His Gln Met Met Ala Gly Ala Asp Val Leu Ala Val Thr Ser Arg
   1               5                   10                  15
   Phe Glu Pro Cys Gly Leu Ile Gln Leu Gln Gly Met Arg Tyr Gly Thr
               20                  25                  30
<210> 7
   <211> 32
   <212> PRT
   <213> Artificial Sequence
<220>
   <223> Description of Artificial Sequence: motif VII
<400> 7
   Ala His Gln Met Met Ala Gly Ala Asp Val Leu Ala Val Thr Ser Arg
   1               5                   10                  15
   Phe Glu Pro Cys Gly Leu Ile Gln Leu Gln Gly Met Arg Tyr Gly Thr
               20                  25                  30
<210> 8
   <211> 32
   <212> PRT
   <213> Artificial Sequence
<220>
   <223> Description of Artificial Sequence: motif VII
<400> 8
   Ala His Met Ile Thr Ala Gly Ala Asp Phe Met Leu Ile Pro Ser Arg
   1               5                   10                  15
   Phe Glu Pro Cys Gly Leu Ile Gln Leu His Ala Met Arg Tyr Gly Thr
               20                  25                  30
<210> 9
   <211> 32
   <212> PRT
   <213> Artificial Sequence
<220>
   <223> Description of Artificial Sequence: motif VII
<400> 9
   Ala His Met Ile Thr Ala Gly Ala Asp Phe Met Leu Val Pro Ser Arg
   1 5 10 15
   Phe Glu Pro Cys Gly Leu Ile Gln Leu His Ala Met Arg Tyr Gly Thr
   20 25 30
<210> 10
   <211> 32
   <212> PRT
   <213> Artificial Sequence
<220>
   <223> Description of Artificial Sequence: motif VII
<400>. 10
<210> 11
   <211> 32
   <212> PRT
   <213> Artificial Sequence
<220>
   <223> Description of Artificial Sequence: motif VII
<400> 11
   Ala His Lys Ile Ile Ala Gly Ala Asp Phe Ile Val Ile Pro Ser Arg
   1 5 10 15
   Phe Glu Pro Cys Gly Leu Val Gln Leu His Ala Met Pro Tyr Gly Thr
   20 25 30
<210> 12
   <211> 32
   <212> PRT
   <213> Artificial Sequence
<220>
   <223> Description of Artificial Sequence: motif VII
<400> 12
   Ala His His Ile Met Ala Gly Ala Asp Leu Leu Ala Val Thr Ser Arg
   1 5 10 15
   Phe Glu Pro Cys Gly Leu Ile Gln Leu Gln Gly Met Arg Tyr Gly Thr
   20 25 30
<210> 13
   <211> 32
   <212> PRT
   <213> Artificial Sequence
<220>
   <223> Description of Artificial Sequence: motif VII
<400> 13
   Ala His His Ile Met Ala Gly Ala Asp Val Leu Ala Val Thr Ser Arg
   1               5                   10                  15
   Phe Glu Pro Cys Gly Leu Ile Gln Leu Gln Gly Met Arg Tyr Gly Thr
               20                  25                  30
<210> 14.
   <211> 32
   <212> PRT
   <213> Artificial Sequence
<220> .
   <223> Description of Artificial Sequence: motif VII
<400> 14
   Ser His Arg Ile Thr Ala Gly Cys Asp Ile Leu Leu Met Pro Ser Arg
   1               5                   10                  15
   Phe Glu Pro Cys Gly Leu Asn Gln Leu Tyr Ala Met Gln Tyr Gly Thr
               20                  25                  30
<210> 15
   <211> 32
   <212> PRT
   <213> Artificial Sequence
<220>
   <223> Description of Artificial Sequence: motif VII
<400> 15
   Ala His Arg Ile Thr Ala Gly Ser Asp Ile Leu Leu Met Pro Ser Arg
   1               5                   10                  15
   Phe Glu Pro Cys Gly Leu Asn Gln Leu Tyr Ala Met Ser Tyr Gly Thr
               20                  25                  30
<210> 16
   <211> 32
   <212> PRT
   <213>.Artificial Sequence
<220>
   <223> Description of Artificial Sequence: motif VII
<400> 16
   Ser His Arg Ile Thr Ala Gly Cys Asp Ile Leu Leu Met Pro Ser Arg
   1               5                   10                  15
   Phe Glu Pro Cys Gly Leu Asn Gln Leu Tyr Ala Met Arg Tyr Gly Thr
               20                  25                  30
<210> 17
   <211> 32
   <212> PRT
   <213> Artificial Sequence
<220>
   <223> Description of Artificial Sequence: motif VII
<400> 17
   Ser His Arg Ile Thr Ala Gly Ala Asp Ile Leu Leu Met Pro Ser Arg
   1               5                   10                  15
   Phe Glu Pro Cys Ala Leu Asn Gln Leu Tyr Ala Met Lys Tyr Gly Thr
               20                  25                  30
<210> 18
   <211> 32
   <212> PRT
   <213> Artificial Sequence
<220>
   <223> Description of Artificial Sequence: motif VII
<400> 18
   Ala His Arg Ile Thr Ala Gly Ala Asp Ile Ala Leu Met Pro Ser Arg
   1               5                   10                  15
   Phe Glu Pro Cys Gly Leu Asn Gln Leu Tyr Ala Met Ala Tyr Gly Thr
               20                  25                  30
<210> 19
   <211> 32
   <212> PRT
   <213> Artificial Sequence
<220>
   <223> Description of Artificial Sequence: motif VII
<400> 19
   Ser His Arg Ile Thr Ala Gly Cys Asp Ile Leu Leu Met Pro Ser Arg
   1 5 10 15
   Phe Glu Pro Cys Gly Leu Asn Gln Leu Tyr Ala Met Gln Tyr Gly Thr
   20 25 30
<210> 20
   <211> 32
   <212> PRT
   <213> Artificial Sequence
<220>
   <223> Description of Artificial Sequence: motif VII
<400> 20
   Ser His Arg Ile Thr Ala Gly Cys Asp Ile Leu Leu Met Pro Ser Arg
   1 5 10 15
   Phe Glu Pro Cys Gly Leu Asn Gln Leu Tyr Ala Met Gln Tyr Gly Thr
   20 25 30
<210> 21
   <211> 32
   <212> PRT
   <213> Artificial Sequence
<220>
   <223> Description of Artificial Sequence: motif VII
<400> 21
   Ala His Arg Ile Thr Ala Gly Ala Asp Val Leu Val Met Pro Ser Arg
   1 5 10 15
   Phe Glu Pro Cys Gly Leu Asn Gln Leu Tyr Ala Met Ala Tyr Gly Thr
   20 25 30
<210> 22
   <211> 32
   <212> PRT
   <213> Artificial Sequence
<220>
   <223> Description of Artificial Sequence: motif VII
<400> 22
   Ala His Arg Ile Thr Ala Gly Ala Asp Ile Leu Leu Met Pro Ser Arg
   1 5 10 15
   Phe Glu Pro Cys Gly Leu Asn Gln Leu Tyr Ala Met Ala Tyr Gly Thr
   20 25 30
<210> 23
   <211> 32
   <212>'PRT
   <213> Artificial Sequence
<220>
   <223> Description of Artificial Sequence: motif VII
<400> 23
   Ala Arg Lys Leu Tyr Ala Ser Ser Asp Phe Ile Leu Met Pro Ser Tyr
    1               5                  10                  15
   Phe Glu Pro Cys Gly Leu Thr Gln Met Ile Gly Met Arg Tyr Gly Cys
               20                  25                  30
<210> 24
   <211> 32
   <212> PRT
   <213> Artificial Sequence .
<220>
   <223> Description of Artificial Sequence: motif VII
<400> 24
   Ala His Gln Ile Tyr Ala Gly Ser Asp Met Phe Leu Met Pro Ser Lys
   1               5                   10                  15
   Phe Glu Pro Cys Gly Leu Thr Gln Leu Tyr Ala Leu Arg Tyr Gly Cys
               20                  25                  30
<210> 25
   <211> 32
   <212> PRT
   <213> Artificial Sequence
<220>
   <223> Description of Artificial Sequence: motif VII
<400> 25
   Ala His Gln Ile Tyr Ala Gly Ala Asp Leu Phe Leu Ile Pro Ser Leu
   1               5                   10                  15
   Phe Glu Pro Cys Gly Leu Ser Gln Met Ile Ala Leu Arg Tyr Gly Thr
               20                  25                  30
<210> 26
   <211> 32
   <212> PRT
   <213> Artificial Sequence
<220>
   <223> Description of Artificial Sequence: motif VII
<400> 26
   Ala His Gln Ile Tyr Ala Gly Ala Asp Leu Phe Leu Ile Pro Ser Leu
   1               5                   10                  15
   Phe Glu Pro Cys Gly Leu Gly Gln Leu Ile Ala Leu Gln Tyr Gly Ala
               20                  25                  30
<210> 27
   <211> 32
   <212> PRT
   <213> Artificial Sequence
<220>
   <223> Description of Artificial Sequence: motif VII
<400> 27
   Ala His Gln Ile Tyr Ala Gly Ala Asp Leu Phe Leu Ile Pro Ser Leu
   1               5                   10                  15
   Phe Glu Pro Cys Gly Leu Gly Gln Leu Ile Ala Leu Gln Tyr Gly Ala
               20                  25                  30
<210> 28
   <211> 32
   <212> PRT
   <213> Artificial Sequence
<220>
   <223> Description of Artificial Sequence: motif VII
<400> 28
   Ser His Leu Met Val Ala Gly Gly Asp Val Ile Leu Val Pro Ser Arg
   1               5                   10                  15
   Phe Glu Pro Cys Gly Leu Thr Gln Leu Tyr Gly Leu Gln Tyr Gly Thr
               20                  25                  30
<210> 29
   <211> 32
   <212> PRT
   <213> Artificial Sequence
<220>
   <223> Description of Artificial Sequence: motif VII
<400> 29
   Ala His Leu Ile Tyr Gly Ala Ala Asp Ile Ile Val Val Pro Ser Asn
   1               5                   10                  15
   Tyr Glu Pro Cys Gly Leu Thr Gln Met Ile Gly Leu Arg Tyr Gly Ala
               20                  25                  30